# EUROPEAN PATENT APPLICATION

(11) **EP 1 605 043 A1**
(43) Date of publication of application: **14.12.2005**
(21) Application number: 04715511.4
(22) Date of filing: 27.02.2004
(51) Int. Cl.: C12N 15/11, C12N 5/10

(54) **INDUCTION OF METHYLATION OF CPG SEQUENCE BY DSRNA IN MAMMALIAN CELL**

(30) Priority: 27.02.2003 US 449860 P
(71) Applicant: National Institute of Advanced Industrial Science and Technology, Tokyo 100-8921 (JP)
(72) Inventor: TAIRA, Kazunari, 3050046 (JP); KAWASAKI, Hiroaki, 1070052 (JP)
(74) Representative: Woods, Geoffrey Corlett
(86) International application number: PCT/JP2004/002448
(87) International publication number: WO 2004/076663

(57) **Abstract**

The present inventors found that in human cells, synthetic small interfering RNAs (siRNAs) targeted to a region containing the CpG islands on gene promoters will specifically induce the methylation of CpG sequences within the target region or adjacent regions. Methylation of the promoters results in suppression at a transcriptional level of expression of genes downstream of the promoters. The present invention provides methods for inducing DNA methylation using the siRNAs, and methods for suppressing gene expression based on siRNA-directed methylation of promoters. The present invention also provides DNA methylation-inducing agents or gene expression-suppressing agents that comprise siRNAs or expression vectors for the siRNAs or dsRNAs.

SEQUENCE LISTING

<110> NATIONAL INSTITUTE OF ADVANCED INDUSTRIAL SCIENCE AND TECHNOLOGY

<120> INDUCTION OF METHYLATION OF CpG SEQUENCE BY dsRNA IN MAMMALIAN CELL

<130> TIR-A0301P

<140> PCT/JP2004/002448
<141> 2004-02-27

<150> US 60/449,860
<151> 2003-02-27

<160> 70

<170> PatentIn version 3.1

<210> 1
<211> 25
<212> RNA
<213> Artificial

<220>
<223> an artificially synthesized target sequence for siRNA

<400> 1

aggccgcucg agcgagagug cagug 25

<210> 2

<211> 25

<212> RNA

<213> Artificial

<220>

<223> an artificially synthesized target sequence for siRNA

<400> 2

gccggugugg uggcacacgc cugua 25

<210> 3

<211> 25

<212> RNA

<213> Artificial

<220>

<223> an artificially synthesized target sequence for siRNA

<400> 3

ccggcaggcg gagguugcag ugagc 25

<210> 4

<211> 25

<212> RNA

<213> Artificial

<220>

<223> an artificially synthesized target sequence for siRNA

<400> 4

acugccccug uccgccccga cuugu 25

<210> 5

<211> 25

<212> RNA

<213> Artificial

<220>

<223> an artificially synthesized target sequence for siRNA

<400> 5

cggcggggcu gggauucgaa cccag 25

<210> 6

<211> 25

<212> RNA

<213> Artificial

<220>

<223> an artificially synthesized target sequence for siRNA

<400> 6

ucaccgcguc uaugcgaggc cgggu 25

<210> 7

<211> 25

<212> RNA

<213> Artificial

<220>

<223> an artificially synthesized target sequence for siRNA

<400> 7

cgggugggcg ggccgucagc uccgc 25

<210> 8

<211>  25

<212>  RNA

<213>  Artificial

<220>

<223>  an artificially synthesized target sequence for siRNA

<400>  8
cgcccugggg aggggucgc gcugc                                                25

<210>  9

<211>  25

<212>  RNA

<213>  Artificial

<220>

<223>  an artificially synthesized target sequence for siRNA

<400>  9
gcgguacggg gggcggugcu ccggg                                               25

<210>  10

<211>  25

<212>  RNA

<213>  Artificial

<220>

<223>  an artificially synthesized target sequence for siRNA

<400>  10
cggugcuccg gggcucaccu ggcug                                                25

<210>  11
<211>  30
<212>  RNA
<213>  Artificial

<220>

<223>  an artificially synthesized target sequence for tRNA-shRNA

<400>  11
uuaucccgga cuccggggga gggggcagag                                           30

<210>  12
<211>  30
<212>  RNA
<213>  Artificial

<220>

<223> an artificially synthesized target sequence for tRNA-shRNA

<400> 12
ugcaggcaac ccagcuuccc ggcgcuagga 30

<210> 13
<211> 30
<212> RNA
<213> Artificial

<220>

<223> an artificially synthesized target sequence for tRNA-shRNA

<400> 13
ccagcuuccc ggcgcuagga gggacgcacc 30

<210> 14
<211> 30
<212> RNA
<213> Artificial

<220>

<223> an artificially synthesized target sequence for tRNA-shRNA

<400> 14

caggccugcg cgaagagagg gagaaaguga 30

<210> 15

<211> 30

<212> RNA

<213> Artificial

<220>

<223> an artificially synthesized target sequence for tRNA-shRNA

<400> 15

ggagggggcg agcugggagc gcgcuugcuc 30

<210> 16

<211> 30

<212> RNA

<213> Artificial

<220>

<223> an artificially synthesized probe sequence

<400> 16

cucugccccc ucccccggag uccgggauaa                                    30

<210>  17
<211>  30
<212>  RNA
<213>  Artificial

<220>
<223>  an artificially synthesized probe sequence

<400>  17
uccuagcgcc gggaagcugg guugccugca                                    30

<210>  18
<211>  30
<212>  RNA
<213>  Artificial

<220>
<223>  an artificially synthesized probe sequence

<400>  18
ggugcguccc uccuagcgcc gggaagcugg                                    30

<210> 19

<211> 30

<212> RNA

<213> Artificial

<220>

<223> an artificially synthesized probe sequence

<400> 19

ggugcguccc uccuagcgcc gggaagcugg 30

<210> 20

<211> 30

<212> RNA

<213> Artificial

<220>

<223> an artificially synthesized probe sequence

<400> 20

gagcaagcgc gcucccagcu cgcccccucc 30

<210> 21

<211> 24

<212> DNA

<213> Artificial

<220>

<223> an artificially synthesized primer sequence

<400> 21

atgggccctt ggagccgcag cctc 24

<210> 22

<211> 24

<212> DNA

<213> Artificial

<220>

<223> an artificially synthesized primer sequence

<400> 22

gagcaattct gcttggattc caga 24

<210> 23

<211>  24

<212>  DNA

<213>  Artificial

<220>

<223>  an artificially synthesized primer sequence

<400>  23
atggagctgg cggccttgtg ccgc                                                24

<210>  24

<211>  25

<212>  DNA

<213>  Artificial

<220>

<223>  an artificially synthesized primer sequence

<400>  24
ttgttcttgt ggaagatgtc cttcc                                               25

<210>  25

<211>  25

<212>  DNA

<213>  Artificial

<220>

<223>  an artificially synthesized primer sequence

<400>  25

atggctcgcg ccaaaacagt catga                                          25

<210>  26

<211>  24

<212>  DNA

<213>  Artificial

<220>

<223>  an artificially synthesized primer sequence

<400>  26

ctcgggactg ggatccatga gaat                                           24

<210>  27

<211>  24

<212>  DNA

<213>  Artificial

<220>

<223>  an artificially synthesized primer sequence

<400>  27

atggggaagg tgaaggtcgg agtc                                              24

<210>  28

<211>  20

<212>  DNA

<213>  Artificial

<220>

<223>  an artificially synthesized primer sequence

<400>  28

tggaatttgc catgggtgga                                                   20

<210>  29

<211>  20

<212>  DNA

<213>  Artificial

<220>

<223>  an artificially synthesized primer sequence

<400> 29
tctagaaaaa ttttttaaaa 20

<210> 30
<211> 20
<212> DNA
<213> Artificial

<220>
<223> an artificially synthesized primer sequence

<400> 30
agagggggtg cgtggctgca 20

<210> 31
<211> 20
<212> DNA
<213> Artificial

<220>
<223> an artificially synthesized primer sequence

<400> 31

cccgggggtc ctggaagcca                                                    20

<210> 32
<211> 21
<212> DNA
<213> Artificial

<220>
<223> an artificially synthesized primer sequence

<400> 32
cccggggggg ctccctggtt t                                                  21

<210> 33
<211> 22
<212> DNA
<213> Artificial

<220>
<223> an artificially synthesized primer sequence

<400> 33
tctagaaaaa ttttttaaaa aa                                                 22

<210> 34

<211> 22

<212> DNA

<213> Artificial

<220>

<223> an artificially synthesized primer sequence

<400> 34

agcctcctga agtgttggat ta 22

<210> 35

<211> 22

<212> DNA

<213> Artificial

<220>

<223> an artificially synthesized primer sequence

<400> 35

acatggtgaa accccgtctt gt 22

<210> 36

<211> 22

<212> DNA

<213> Artificial

<220>

<223> an artificially synthesized primer sequence

<400> 36

tgtctcagcc tattgagtag ct 22

<210> 37

<211> 22

<212> DNA

<213> Artificial

<220>

<223> an artificially synthesized primer sequence

<400> 37

taggctgaga caggagagtc tc 22

<210> 38

<211> 22

<212> DNA

<213> Artificial

<220>

<223> an artificially synthesized primer sequence

<400> 38

ccaggctgga gtgcagtggc ac 22

<210> 39

<211> 22

<212> DNA

<213> Artificial

<220>

<223> an artificially synthesized primer sequence

<400> 39

ggcaatacag ggagacacag cg 22

<210> 40

<211> 22

<212>  DNA

<213>  Artificial

<220>

<223>  an artificially synthesized primer sequence

<400>  40

acaccaccac gccaggctaa tt                                                22

<210>  41

<211>  22

<212>  DNA

<213>  Artificial

<220>

<223>  an artificially synthesized primer sequence

<400>  41

ttctgatccc aggtcttagt ga                                                22

<210>  42

<211>  22

<212>  DNA

<213>  Artificial

<220>

<223>  an artificially synthesized primer sequence

<400>  42
taggtgggtt atgggacctg ca                                                22

<210>  43
<211>  22
<212>  DNA
<213>  Artificial

<220>
<223>  an artificially synthesized primer sequence

<400>  43
agcaactcca ggctagaggg tc                                                22

<210>  44
<211>  22
<212>  DNA
<213>  Artificial

<220>

<223>  an artificially synthesized primer sequence

<400>  44
gcgcggaccc ctccccaggg cg                                                22

<210>  45
<211>  22
<212>  DNA
<213>  Artificial

<220>
<223>  an artificially synthesized primer sequence

<400>  45
ggctagaggg tcaccgcgtc ta                                                22

<210>  46
<211>  20
<212>  DNA
<213>  Artificial

<220>
<223>  an artificially synthesized primer sequence

<400> 46
taccgctgat tggctgaggg 20

<210> 47
<211> 21
<212> DNA
<213> Artificial

<220>
<223> an artificially synthesized primer sequence

<400> 47
cgggtgggcg ggccgtcagc t 21

<210> 48
<211> 22
<212> DNA
<213> Artificial

<220>
<223> an artificially synthesized primer sequence

<400> 48
attggctgag ggttcacctg cc 22

<210> 49

<211> 22

<212> DNA

<213> Artificial

<220>

<223> an artificially synthesized primer sequence

<400> 49

ggcaggtgaa ccctcagcca at 22

<210> 50

<211> 22

<212> DNA

<213> Artificial

<220>

<223> an artificially synthesized primer sequence

<400> 50

tgcgtggctg cagccaggtg ag 22

<210> 51

<211> 22

<212> DNA

<213> Artificial

<220>

<223> an artificially synthesized primer sequence

<400> 51

ggcaggtgaa ccctcagcca at 22

<210> 52

<211> 22

<212> DNA

<213> Artificial

<220>

<223> an artificially synthesized primer sequence

<400> 52

tgcgtggctg cagccaggtg ag 22

<210> 53

<211> 22

<212>  DNA

<213>  Artificial

<220>

<223>  an artificially synthesized primer sequence

<400>  53

caggaaagtt taagataaaa cc                                                22

<210>  54

<211>  22

<212>  DNA

<213>  Artificial

<220>

<223>  an artificially synthesized primer sequence

<400>  54

ctcggagaat ccctaaatgc ag                                                22

<210>  55

<211>  22

<212>  DNA

<213>  Artificial

<220>

<223>  an artificially synthesized primer sequence

<400>  55
cgaggaaaag tgtgagaacg gc                                                22

<210>  56
<211>  22
<212>  DNA
<213>  Artificial

<220>
<223>  an artificially synthesized primer sequence

<400>  56
cgcgcaggcc tgggtgcgtc cc                                                22

<210>  57
<211>  22
<212>  DNA
<213>  Artificial

<220>

<223>  an artificially synthesized primer sequence

<400>  57
cgaggaaaag tgtgagaacg gc                                                22

<210>  58
<211>  20
<212>  DNA
<213>  Artificial

<220>
<223>  an artificially synthesized primer sequence

<400>  58
caggcctggg tgcgtccctc                                                   20

<210>  59
<211>  20
<212>  DNA
<213>  Artificial

<220>
<223>  an artificially synthesized primer sequence

<400>  59

gagggacgca cccaggcctg                                                    20

<210>  60

<211>  22

<212>  DNA

<213>  Artificial

<220>

<223>  an artificially synthesized primer sequence

<400>  60

ctgggagtgg caactcccag ct                                                 22

<210>  61

<211>  20

<212>  DNA

<213>  Artificial

<220>

<223>  an artificially synthesized primer sequence

<400>  61

agacttgttg gaatgcagtt                                                    20

<210> 62

<211> 22

<212> DNA

<213> Artificial

<220>

<223> an artificially synthesized primer sequence

<400> 62

cttcattctt atacttcctc aa 22

<210> 63

<211> 24

<212> DNA

<213> Artificial

<220>

<223> an artificially synthesized primer sequence

<400> 63

gtcttcacca ccatggagaa ggct 24

<210> 64

<211> 24

<212> DNA

<213> Artificial

<220>

<223> an artificially synthesized primer sequence

<400> 64

gccatccaca gtcttctggg tggc 24

<210> 65

<211> 22

<212> RNA

<213> Artificial

<220>

<223> an artificially synthesized siRNA sequence

<220>

<221> misc_feature

<222> (21)..(22)

<223> n indicates dT

<400> 65

aagcaugagc accguucucc nn                                                22

<210>  66

<211>  22

<212>  RNA

<213>  Artificial

<220>

<223>  an artificially synthesized siRNA sequence

<220>

<221>  misc_feature

<222>  (21)..(22)

<223>  n indicates dT

<400>  66

ggagaacggu gcucaugcuu nn                                                22

<210>  67

<211>  22

<212>  RNA

<213>  Artificial

<220>

<223>  an artificially synthesized siRNA sequence

<220>

<221>  misc_feature

<222>  (21)..(22)

<223>  n indicates dT

<400>  67

aagcuugugc agcguugucc nn                                                22

<210>  68

<211>  22

<212>  RNA

<213>  Artificial

<220>

<223>  an artificially synthesized siRNA sequence

<220>

<221>  misc_feature

<222>  (21)..(22)

<223>  n indicates dT

<400>  68

ggacaacgcu gcacaagcuu nn                                                22

<210> 69

<211> 21

<212> DNA

<213> Artificial

<220>

<223> an artificially synthesized siRNA sequence

<400> 69

tatggaatat tatcttgtaa a 21

<210> 70

<211> 21

<212> DNA

<213> Artificial

<220>

<223> an artificially synthesized siRNA sequence

<400> 70

tttacaagat aatattccat a 21

## Description

### Technical Field

The present invention relates to methods for methylating CpG sequences in DNAs using dsRNAs, and relates to methylation-inducing agents that comprise dsRNAs. The present invention also relates to methods for using dsRNAs to suppress gene expression by inducing the methylation of CpG sequences in promoter regions, and relates to gene expression-suppressing agents that comprise dsRNAs.

### Background Art

Double-stranded RNAs (dsRNAs) induce post-transcriptional gene silencing through RNA interference (RNAi) in animals and plants (Fire A. *et al*. Nature 391, 806-811 (1998); Hammond, S.M. *et al*. Nature Rev. Genet. 2, 110-119 (2001); Hutvagner, G., & Zamore, P.D. Curr. Opin. Genet. Dev. 12, 225-232 (2002)). In this system, small interference RNAs (siRNAs) produced by RNaseIII Dicer are incorporated into RNAi-induced silencing complexes (RISCs) (Tuschl, T. *et al.* Genes Dev., 13, 3191-3197 (1999); Hammond, S.M. *et al.* Nature, 404, 293-296 (2000); Zamore, P. *et al.* Cell 101, 25-33 (2000); Bernstein, E. *et al.* Nature 409, 363-366 (2001); Elbashir, S.M. *et al.* Genes Dev. 15, 188-200 (2001)). The siRNA-RISC complexes thus obtained promote mRNA degradation in the cytoplasm (Tuschl, T. *et al.* Genes Dev., 13, 3191-3197 (1999); Hammond, S.M. *et al.* Nature, 404, 293-296 (2000); Elbashir, S.M. *et al.* Nature, 411, 494-498 (2001); Hutvagner, G, & Zamore, P.D. Science 297, 2056-2060 (2002); Zeng, Y., & Cullen, B.R. RNA, 8, 855-860 (2002); Kawasaki, H., & Taira, K. Nucleic Acids Res. 31, 700-707 (2003)). It has also been reported that, in plants, dsRNAs can induce sequence-specific DNA methylation and regulate gene expression at the transcriptional level (Pelissier, T., & Wassenegger, M. RNA 6, 55-65 (2000); Mette, M.F. *et al*. EMBO J. 19, 5194-201 (2000); Vaucheret, H., & Fagard, M. Trends Genet. 17, 29-35 (2001); Jones, L. *et al.* Curr. Biol. 11, 747-757 (2001); Aufsatz, W. *et al.* Proc. Natl. Acad. Sci. USA 99, 16499-16506 (2002); Hamilton, A. *et al.* EMBO J. 21, 4671-4769 (2002); Aufsatz, W. *et al.* EMBO J. 21, 6832-6841 (2002)). In other words, both long and short dsRNAs can induce sequence-specific DNA methylation, namely RNA-directed DNA methylation (RdDM), in plants. Furthermore, introduced genes can also induce sequence-specific DNA methylation (Vaucheret, H., & Fagard, M. Trends Genet. 17, 29-35 (2001); Morel, J.B. *et al.* Curr. Biol. 10, 1591-1594 (2000); Beclin, C. *et al.* Curr. Biol. 12, 684-688 (2002)). These systems have been thought to serve as defense systems against RNA and DNA viruses. However, it was unclear whether such mechanisms existed in mammalian cells. Further studies are required to clarify whether defense systems related to the above-described systems exist in human cells via RMDM or introduced genes.

The DNA methylation of gene promoters has an important role in inducing tumor genesis and development by regulating the expression of specific genes (Li, E. Nature Rev. Genet. 3, 662-673 (2002); Esteller, M. Oncogene 21, 5427-5440 (2002)). Many proteins involved in DNA methylation, such as proteins belonging to the families MeCP2, MBD, and DMNT, have been identified and well characterized in human cells. However, it remains unclear as to how and by what these proteins are directed, as well as how the methylatation of specific CpG target sites of cognate genes is induced during tumor genesis or development. Synthetic siRNAs and tRNA vector-based siRNAs are localized predominantly in the cytoplasm, where siRNA-mediated degradation of mRNAs also occurs. One possibility is that a small fraction of siRNA-protein complexes might be transported to the nucleus. Alternatively, siRNAs might gain access to genomic DNAs during cell division, since the nuclear membrane disappears at this time.

### Disclosure of the Invention

The present inventors demonstrated that both synthetic small interfering RNAs (siRNA) and vector-based siRNAs induce sequence-specific DNA methylation in human cells. Synthetic siRNAs (E-cadherin-siRNAs) targeted to CpG islands on E-cadherin promoters induced significant DNA methylation in MCF-7 cells. As a result, these siRNAs suppressed the expression of the E-cadherin gene at the transcriptional level. In addition, E-cadherin-siRNA-directed DNA methylation disappeared on the disruption of DNMT1 by specific siRNAs. Furthermore, vector-based siRNAs targeted to the erbB2 promoter also induced DNA methylation and transcriptional gene silencing in MCF-7 cells. Thus, in human cells, siRNAs targeted to CpG islands on the promoters of genes of interest can induce gene silencing at the transcriptional level by means of DNMT1-dependent DNA methylation, and may result in new types of gene therapeutic agents. The present invention is based on the above findings, and specifically includes:
[1] A DNA methylation-inducing agent that comprises a dsRNA targeted to a site comprising CpG or CpNG (where N is any one of A, T, C, and G) in a DNA in a mammalian cell.
[2] A DNA methylation-inducing agent that comprises an expression vector comprising a DNA encoding a dsRNA targeted to a site comprising CpG or CpNG (where N is any one of A, T, C, and G) in a DNA in a mammalian cell.
[3] The DNA methylation-inducing agent of [1] or [2], wherein the dsRNA consists of 30 nucleotides or less.
[4] The DNA methylation-inducing agent of [1] or [2], wherein the dsRNA consists of 30 to 5,000 nucleotides.
[5] The DNA methylation-inducing agent of any one of [1] to [4], wherein the dsRNA comprises a hairpin structure.
[6] The DNA methylation-inducing agent of [2], wherein the vector further encodes Dicer.
[7] The DNA methylation-inducing agent of [2], wherein an exon-intron-exon cassette is operably linked downstream of a promoter and the DNA encoding the dsRNA is inserted into an intron.
[8] The DNA methylation-inducing agent of [7], wherein the exon-intron-exon cassette is derived from an immunoglobulin gene.
[9] The DNA methylation-inducing agent of any one of [2] to [7], wherein the DNA encoding the dsRNA is operatively linked to a PolI or PolII promoter in the expression vector.
[10] The DNA methylation-inducing agent of [9], wherein the PolIII promoter is any one of tRNA promoter, U6 promoter, and H1 promoter.
[11] The DNA methylation-inducing agent of [10], wherein the PolII promoter is any one of SV40 promoter, CMV promoter, and SRα promoter.
[12] The DNA methylation-inducing agent of any one of [2] to [11], wherein the expression vector is a viral expression vector.
[13] The DNA methylation-inducing agent of any one of [2] to [12], wherein the viral expression vector is any one of retrovirus, adenovirus, and lentivirus.
[14] The DNA methylation-inducing agent of any one of [2] to [13], wherein the DNA encoding the dsRNA is operatively linked to a promoter comprising a tetracycline operator sequence (TetO) in the expression vector.
[15] A DNA methylation method that comprises the step of introducing the methylation-inducing agent of any one of [1] to [14] into a mammalian cell.
[16] The DNA methylation method of [15], wherein the mammal is human.
[17] A gene expression-suppressing agent that comprises a dsRNA targeted to a site comprising CpG or CpNG (wherein, N is any one of A, T, C, and G) in a gene promoter in a mammalian cell.
[18] A gene expression-suppressing agent that comprises an expression vector encoding a dsRNA targeted to a site comprising CpG or CpNG (where N is any one of A, T, C, and G) in a gene promoter in a mammalian cell.
[19] The gene expression-suppressing agent of [17] or [18], wherein the types of dsRNA differ and the each of the dsRNAs target a different site comprising CpG or CpNG (where N represents any one of A, T, C, and G).
[20] The gene expression-suppressing agent of any one of [17] to [19], wherein the dsRNA consists of 30 nucleotides or less.
[21] The gene expression-suppressing agent of any one of [17] to [19], wherein the dsRNA consists of 30 to 5,000 nucleotides.
[22] The gene expression-suppressing agent of any one of [17] to [21], wherein the dsRNA comprises a hairpin structure.
[23] The gene expression-suppressing agent of [18], wherein the vector further encodes Dicer.
[24] The gene expression-suppressing agent of [18], wherein an exon-intron-exon cassette is operably linked downstream of a promoter and the DNA encoding the dsRNA is inserted into the intron.
[25] The gene expression-suppressing agent of [18], wherein the exon-intron-exon cassette is derived from an immunoglobulin gene.
[26] The gene expression-suppressing agent of any one of [18] to [25], wherein the DNA encoding the dsRNA is operatively linked to a PolI or PolII promoter in the expression vector.
[27] The gene expression-suppressing agent of [26], wherein the PolIII promoter is any one of tRNA promoter, U6 promoter, and H1 promoter.
[28] The gene expression-suppressing agent of [26], wherein the PolII promoter is any one of SV40 promoter, CMV promoter, and SRα promoter.
[29] The gene expression-suppressing agent of any one of [18] to [28], wherein the expression vector is a viral expression vector.
[30] The gene expression-suppressing agent of [29], wherein the viral expression vector is any one of retrovirus, adenovirus, and lentivirus.
[31] The gene expression-suppressing agent of any one of [18] to [30], wherein the DNA encoding the dsRNA is operatively linked to a promoter comprising a tetracycline operator sequence (TetO) in the expression vector.
[32] The gene expression-suppressing agent of [17] to [31], wherein the gene is a disease-associated gene whose expression is involved in a disease.
[33] The gene expression-suppressing agent of [32], wherein the disease is a tumor.
[34] The gene expression-suppressing agent of [33], wherein the gene is erbB2.
[35] A cell proliferation-suppressing agent that comprises the gene expression-suppressing agent of [34] as an active ingredient.
[36] A gene expression-suppressing method that comprises the step of introducing the gene expression-suppressing agent of any one of [18] to [34] into a cell.
[37] The gene expression-suppressing method of [36] that further comprises the step of introducing dsRNAs targeted to a coding region of a gene into a cell.
[38] The gene expression-suppressing method of [36] or [37], wherein the mammalian cell is derived from a human.

In the first embodiment, the present invention provides methylation-inducing agents that use dsRNA for methylating CpG or CpNG sequences in a sequence-specific manner in mammalian cells. The methylation-inducing agents of the present invention comprise dsRNAs targeted to DNA sites comprising CpG or CpNG sequences in mammalian cells, or vectors encoding the dsRNAs.

In the present invention, the "CpG or CpNG in DNAs" may be any CpG or CpNG sequences selected from those in DNAs to be methylated. Methylation has the function of regulating various biological activities *in vivo.* For example, methylating CpG sequences or such in promoter regions suppresses downstream gene expression. Thus, in the present invention, CpG or CpNG sequences in the promoter regions of mammalian genes can be preferably selected. Herein, the "N" of CpNG represents any of A, G, C, and T.

The "sites" of the present invention may comprise at least one unit of CpG or CpNG, and preferably, a CpG island comprising two or more CpG units can be selected as the site. By using a site comprising two or more CpG units as a target, two or more methyl groups can be introduced into the site. This can enhance the biological effects of methylation.

The phrase "dsRNAs targeted to" refers to dsRNAs that are complementary to double-stranded DNAs of the above-described CpG- or CpNG-comprising sites. More specifically, the term refers to double-stranded RNAs formed by pairing two single-stranded RNAs complementary to the (+) and (-) strands of an above-described site comprising CpG, where the double-stranded DNAs consist of complementary (+) and (-) strands. The term "targeted" preferably refers to dsRNAs formed by pairing RNA strands each complementary to a double-stranded DNA site comprising CpG or CpNG sequences to which methyl groups are to be introduced. However, as described in the Examples, methyl groups can be also introduced into CpG or CpNG sequences that are near sites corresponding to the dsRNAs. Thus, the phrase "dsRNAs targeted to" also includes not only dsRNAs directly corresponding to the "DNA sites comprising CpG or CpNG sequences" as described above, but also includes dsRNAs complementary to DNA sequences not corresponding to sites to which methyl groups are to be introduced. The distance between the dsRNAs and the DNA sequence sites to which methyl groups are to be introduced may range, for example, from about 50 to 60 nucleotides. In other words, CpG or CpNG sequences as the targets can be methylated by using dsRNAs complementary to a position about 50 to 60 nucleotides away from the site comprising the CpG or CpNG to which the methyl groups are to be introduced. As described below, the target DNA sequences are not necessarily perfectly complementary to the RNA sequences, and either or both of the strands of a dsRNA may contain non-complementary nucleotides.

Any dsRNA constructs used for RNA interference (RNAi) can be used as the "dsRNAs (double-stranded RNAs)" of the present invention. The present inventors found that dsRNAs for RNAi, which have been widely used in recent years, induced DNA methylation in mammalian cells. The present inventors then developed DNA-methylation agents for mammals based on this finding. At present, dsRNAs for RNAi in mammalian cells include both artificially synthesized dsRNAs, and dsRNAs expressed in mammalian cells using expression vectors. Thus, both types of dsRNAs can also be used as the methylation-inducing agents of the present invention.

The cytotoxicity of long dsRNAs to mammalian cells must be considered in cases where artificially synthesized dsRNAs are introduced into mammalian cells. Thus, short dsRNAs are preferably used as the artificially synthesized dsRNAs (hereinbelow referred to as "synthetic dsRNAs"). The short dsRNAs may range, for example, from 15 to 49 base pairs, preferably from 15 to 35 base pairs, and more preferably from 21 to 30 base pairs.

Further, dsRNAs produced from expression vectors (hereinbelow also referred to as "vector-based dsRNAs" or the like) are also preferably short dsRNAs in consideration of cytotoxicity. However, long dsRNAs can be expressed by using constructs designed to produce short dsRNAs by cleavage of the long dsRNAs immediately after expression. The cytotoxicity is caused by enzymes that work to eliminate long dsRNAs when they invade the cytoplasm. Thus, not only short dsRNAs but also long dsRNAs can be used when directly expressed in a nucleus in which genomic DNAs exist. Accordingly, vector-based dsRNAs may range, for example, from 15 to 50,000 base pairs, preferably from 21 to 10,000 base pairs, and more preferably from 30 to 5,000 base pairs. The short dsRNAs described above are preferably used to methylation of specific target CpGs or CpNGs. In contrast, the long dsRNAs are suited to methylate CpGs or CpNGs in a greater range of DNAs.

An example of a construct capable of expressing long dsRNAs using expression vectors, and then cleaving them to short dsRNAs immediately after expression, is a construct for expressing enzymes such as Dicer, which can cleave long dsRNAs to short dsRNAs simultaneously on expression of the long dsRNAs. More specifically, such an expression vector comprises DNA encoding long dsRNAs and the Dicer gene. When both are simultaneously expressed in cells, Dicer can cleave the long dsRNAs to short dsRNAs immediately after their expression. More specific constructs can be prepared with reference to the Examples herein below.

An exemplary construct for using expression vectors to directly express dsRNAs in a nucleus comprising genomic DNAs is a construct in which DNAs encoding dsRNAs are inserted into an intron in an expression cassette comprising an exon-intron-exon. Eukaryotic introns are excised from RNA precursors by processing in the nucleus. Therefore, when expression cassettes comprising the above exon-intron-exon are expressed in mammalian cells, the introns are excised from the RNA precursors in the nucleus, and the dsRNAs inserted into the introns induce methylation at target sites in the genomic DNA, without activating cytoplasmic enzymes and others. In the Examples described below, it was indeed confirmed that long dsRNAs expressed using an expression cassette comprising an exon-intron-exon induced DNA methylation, but produced no cytotoxic response.

An expression cassette comprising an exon-intron-exon may be derived from any gene, as long as the expression products do not have negative effects in cells and do not inhibit the DNA methylation that is an objective of the present invention. The "exon-intron-exon" units that can be used in the present invention include, for example, cdk inhibitor p16 gene, Bcl2 gene, and the like, and preferable examples are exon-intron-exon units derived from immunoglobulins. The exon-intron-exon unit may contain at least one intron flanked by two exons. Units comprising two or more introns, such as exon-(intron-exon-intron)ₙ-exon (wherein, n represents an integer of one or greater) for example, may also be used. By using units comprising introns encoding dsRNAs targeted to different DNA sites, two or more sites can be simultaneously methylated.

Artificially synthesized dsRNAs and dsRNAs expressed using a vector may or may not have a hairpin structure with a loop at one end (sometimes referred to as a "stem-loop structure").

The two loopless ends of dsRNAs that do not have loop at their ends may be blunt or sticky (protruding) ends. The structures of sticky (protruding) ends include not only the structures with a 3' protruding end reported for siRNA (Elbashir, S. M *et al*. Duplexes of 21-nucleotide RNAs mediate RNA interference in cultured mammalian cells. Nature 411, 494-498 (2001); Caplen, N. J. *et al*. Specific inhibition of gene expression by small double-stranded RNAs in invertebrate and vertebrate systems. Proc Natl Acad Sci USA 98, 9742-9747 (2001)), but also structures with a 5' protruding end, as long as they induce DNA methylation. The number of protruding nucleotides is not limited to two to three as reported in the above references, and may be any number of nucleotides, as long as an RNAi effect can be induced. The number of nucleotides may range, for example, from one to eight, and preferably from two to four.

The term "dsRNAs having a hairpin structure" refers to dsRNAs comprising a stem-loop structure, which is linked to one end of a double-stranded RNA by a linker RNA or the like. The length of hairpin dsRNAs is based on the double-stranded RNA portion (the stem portion). Thus, when measured as the length of the double-stranded RNA portion (the stem portion), synthetic dsRNAs may range, for example, from 15 to 49 base pairs, preferably from 15 to 35 base pairs, and more preferably from 21 to 30 base pairs; while vector-based dsRNAs may range, for example, from 15 to 50,000 base pairs, preferably from 21 to 10,000 base pairs, and more preferably from 30 to 5,000 base pairs, as described above.

The linker lengths are not limited, as long as they are a length that does not inhibit stem portion pairing. For example, a cloverleaf tRNA structure may be used as a linker portion to stabilize stem portion pairing and to suppress recombination between DNAs encoding the stem portion. Alternatively, even when the length of a linker inhibits stem portion pairing, the construct can comprise a structure such that, for example, an intron is included in the linker portion, the intron is excised during the processing of precursor RNAs to mature RNAs, and stem portion pairing is thus made possible.

The double-stranded RNA portions formed by RNA pairing in dsRNAs include not only perfectly complementary double-stranded RNAs, but also double-stranded RNAs containing unpaired portions such as mismatches (where corresponding nucleotide are not complementary) and bulges (where one strand has no corresponding nucleotides), in their sense RNA strand. A double-stranded RNA may comprise such unpaired portions as long as they inhibit neither the formation of dsRNAs, nor the induction of methylation.

"Bulges" as described above preferably consist of one to two unpaired nucleotides, and the number of bulges varies depending on the length of dsRNAs. For example, for dsRNAs comprising about 30 nucleotides, the sense RNA strands comprise one to seven bulges, and preferably about one to five bulges, within the double-stranded RNA region. The number of the above-described "mismatches" also varies depending on the length of the dsRNA. For example, for dsRNAs comprising about 30 nucleotides, the sense RNA strand comprises one to seven mismatches, and preferably about one to five mismatches, in the double-stranded RNA region. dsRNAs may comprise both bulges and mismatches. In such cases, the number of bulges and mismatches ranges, for example, from one to seven in total, and preferably from about one to five in total.

As described above, the structure of vector-based dsRNAs may or may not have a hairpin structure at one end. When dsRNAs with two loopless ends are expressed using vectors, the DNAs encoding each strand (hereinbelow, one strand is called the "sense RNA strand" and the other is called the "antisense RNA strand") of the dsRNAs are placed in tandem in the vectors, and promoters and terminators are respectively arranged upstream and downstream of each DNA. By introducing vectors constructed as described above into cells, each of the RNA strands are expressed and annealed to form dsRNAs in the cells.

Meanwhile, expression vectors for hairpin dsRNAs can be constructed by constructing units in which DNAs encoding antisense and sense strands are ligated with inverse orientations via DNAs encoding linker RNAs (linker DNAs). The units comprise "sense RNA strand - loop - antisense RNA strand", preferably in this order. A promoter can be ligated at one end of such units to construct the expression vectors for hairpin dsRNAs. There is no limitation on the length and sequence of linker DNAs used to construct the expression vectors for hairpin dsRNA, as long as the sequence is not a termination sequence or the like which inhibits the formation of siRNAs, and as long as the sequence and the length of the linker allows pairing of the stem portion of the mature RNAs. For example, the DNAs encoding tRNAs as described above can be used as the linker DNAs.

As the promoters for expressing these dsRNAs, PolII and PolIII promoters can be preferably used. PolIII promoters include, for example, U6 promoters, tRNA promoters, adenovirus VA1 promoter, 5S rRNA promoter, 7SK RNA promoter, 7SL RNA promoter, and H1 RNA promoter. When a U6 promoter is used, four uridine residues are added to the 3' end of the RNAs; however, the 3' protruding ends of the siRNAs ultimately produced can be freely adjusted to be four, three, two, one, or zero nucleotides by adding zero, one, two, three, or four adenines to the antisense-coding DNAs and sense-coding DNAs. When other promoters are used, the number of protruding nucleotides can also be freely adjusted by the same procedure.

When PolIII promoters are used, terminators are preferably added at the 3' ends of sense-coding DNAs and antisense-coding DNAs, to express only short RNAs and appropriately terminate the transcription. The type of terminator is not limited, as long as its sequence allows the termination of transcription driven by the promoters. For example, a sequence comprising four or more consecutive Ts (thymine) or As (adenine), or a sequence that can form a palindromic structure, can be used as the terminator. Sequences comprising four or more consecutive Ts (thymine) are especially preferred.

PolII promoters include retroviral LTR promoters, cytomegalovirus promoters, T7 promoters, T3 promoters, SP6 promoters, RSV promoters, EF-1α promoters, β-actin promoters, γ-globulin promoters, SRα promoters, and others. These PolII promoters are preferably used to express moderately long dsRNAs. When generating short dsRNAs using a PolII promoter, it is preferable to also use a means for RNA cleavage by self-processing, such as a ribozyme. Antisense or sense RNAs of interest can be produced by the combined use of the means for RNA cleavage by self-processing to trim moderately long RNA strands expressed by the PolII promoter. Stem-loop RNAs can be also generated by inserting a stem-loop sequence immediately downstream of the PolII promoter, and placing a polyA addition signal downstream of the sequence. Such ribozyme-based units for generating antisense or sense RNAs can be constructed, for example, with reference to WO 03/046186. Ribozymes with self-processing activity include hammerhead ribozymes, hairpin ribozymes, HDV ribozymes, and ribozymes derived from *Tetrahymena* (Biochem. Biophys. Res. Commu n., Vol.186, pp.1271-1279 (1992); Proc. Natl. Aca d. Sci. USA, Vol.90, pp.11302-11306 (1993); BIO medica, Vol.7, pp.89-94 (1992); Gene, Vol.122, pp.85-90 (1992)).

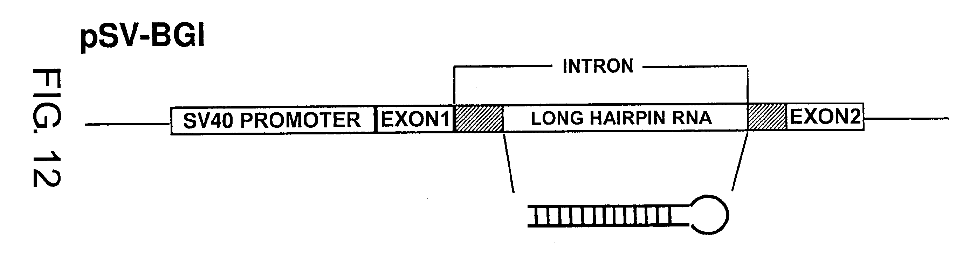

siRNAs can be expressed at a preferred time by using an inducible promoter as the above-described promoter. Such inducible promoters include tRNA promoters and U6 promoters to which a tetracycline operator (TetO) sequence is added to allow transcriptional induction by tetracyclirie (Ohkawa, J. & Taira, K. Control of the functional activity of an antisense RNA by a tetracycline-responsive derivative of the human U6 snRNA promoter. Hum Gene Ther. 11, 577-585 (2000); Fig. 12). Alternatively, tissue-specific DNA methylation can be induced in a tissue by expressing dsRNAs in a tissue-specific manner, using a tissue-specific promoter or a DNA recombination system such as the Cre-LoxP system. Expression systems for dsRNAs using DNA recombination systems such as the Cre-LoxP system can be constructed with reference to WO 03/046186, as described above.

"Vectors" of the present invention may be selected appropriately according to the type of cells into which the vectors are to be introduced. For example, when mammalian cells are used, the vectors may include, but are not limited to, viral vectors such as retroviral vectors, adenoviral vectors, adeno-associated viral vectors, vaccinia viral vectors, lentiviral vectors, herpes viral vectors, alpha viral vectors, EB viral vectors, papilloma viral vectors, and foamy viral vectors; and non-viral vectors such as cationic liposomes, ligand-DNA complexes, and gene guns (Y. Niitsu *et al*., Molecular Medicine 35: 1385-1395 (1998)). Dumbbell DNAs (Zanta M.A. *et al*., Gene delivery: a single nuclear localization signal peptide is sufficient to carry DNA to the cell nucleus. Proc Natl Acad Sci USA. 1999 Jan 5;96(1):91-6), nuclease-resistant modified DNAs, and naked plasmids can also be preferably used in addition to viral vectors (Liu F, Huang L. Improving plasmid DNA-mediated liver gene transfer by prolonging its retention in the hepatic vasculature. J. Gene Med. 2001 Nov-Dec;3(6):569-76).

Each of the RNA strands of a vector-based dsRNA is not necessarily expressed from the same vector. The dsRNAs may be designed so that the antisense and sense RNAs are expressed from different vectors.

In the second embodiment, the present invention provides methods for methylating DNAs. The methylation methods of the present invention comprise the step of introducing mammalian cells with vectors directing the expression of dsRNAs or dsRNAs that serve as the above-described DNA methylation-inducing agents.

The type of mammalian cell is not particularly limited, and the cells may be derived from humans, monkeys, dogs, mice, rats, rabbits, etc. There is also no particular limitation on the type of method for introducing dsRNAs or vectors into such mammalian cells. The methods can be selected from calcium phosphate methods (Virology, Vol. 52, p. 456 (1973)), electroporation (Nucleic Acids Res., Vol. 15, p. 1311 (1987)), lipofection methods (J. Clin. Biochem. Nutr., Vol. 7, p. 175 (1989)), viral infection-mediated introduction methods (Sci. Am., p. 34, March (1994)), gene gun methods, and others. Methods for introduction into plant cells include electroporation (Nature, Vol. 319, p. 791 (1986)), polyethylene glycol methods (EMBO J., Vol. 3, p. 2717 (1984)), particle gun methods (Proc. Natl. Acad. Sci. USA, Vol. 85, p. 8502 (1988)), *Agrobacterium*-mediated introduction methods (Nucleic. Acids Res., Vol. 12, p. 8711 (1984)), and the like. Alternatively, for convenience dsRNAs or vectors may be introduced using commercially-available kits, as described in the Examples.

The methylation can be confirmed by methylation-specific PCR analysis or bisulfite sequencing methods, as in the Examples. The specific procedures for these analyses are described in the Examples. When long dsRNAs are used, their cytotoxicity can be evaluated according to elfα activity, which is described in detail in the Examples.

In cells into which the above-described DNA methylation-inducing agents are introduced, DNAs are methylated in a sequence-specific manner. Herein, the term "sequence-specific manner" refers to the methylation of CpG or CpNG in target sites for dsRNAs. The findings in the Examples suggest that histone H3 methylation and DNA methyltransferase 1 are involved in dsRNA-directed DNA methylation.

DNA methylation is a mechanism for controlling various biological activities. Thus, the above-described DNA methylation-inducing agents and the methods for inducing DNA methylation are useful to elucidate *in vivo* mechanisms regulated by methylation.

In the third embodiment, the present invention provides agents for suppressing gene expression, and gene expression methods using these agents.

DNA methylation is a mechanism for regulating gene expression. Specifically, the methylation of CpG islands in gene promoter regions causes an alteration in DNA conformation, resulting in suppression of gene expression. Thus, gene expression can be suppressed by using the above methylation-inducing agents to methylate CpG islands in gene promoter regions. Unlike siRNAs, which suppress the translation of mRNAs into proteins, the gene expression-suppressing agents of the present invention suppress the transcription of DNAs into mRNAs. Accordingly, the gene expression-suppressing agents of the present invention can induce transcriptional silencing (or gene knockdown at a transcriptional step).

When the above-described methylation-inducing agents are used as gene expression-suppressing agents, dsRNAs, or vectors encoding the dsRNAs, are constructed to target a site comprising a CpG or CpNG (where N is any one of A, T, C, and G) of a gene promoter in a mammalian cell. Furthermore, the greater the degree of methylation by the gene-expression suppressing agents, the more effectively the expression of genes is suppressed. Thus, the number of target sites is preferably not one, but two or more. When the above-described methylation-inducing agents target two or more sites, they preferably use a combination of dsRNAs targeting different sites, and these dsRNAs can be the above-described vector-based long dsRNAs (for example, dsRNAs derived from a co-expression system for long dsRNAs and Dicer, or a system where DNAs encoding dsRNAs are inserted into the intron of an exon-intron-exon) or short dsRNAs.

As described above, the gene expression-suppressing agents of the present invention suppress expression at a transcriptional level. Thus, for more intensive suppression of gene expression, dsRNAs for RNAi may be used in combination with the gene expression-suppressing agents of the present invention. Specifically, gene expression-suppressing agents may be prepared by combining dsRNAs (siRNAs) targeted to a coding region and dsRNAs targeted to CpG islands of a gene promoter region. By using siRNAs combined with the gene expression-suppressing agents of the present invention as described above, the gene expression-suppressing agents of the present invention will suppress gene expression at a transcriptional level, and even if mRNAs are transcribed, the siRNAs will suppress their translation. Thus, strong gene-knockdown agents can be prepared by combining the gene expression-suppressing agents of the present invention with siRNAs that target identical genes.

Genes whose expressions are to be suppressed according to the present invention are not particularly limited, and may be any genes as long as they comprise a CpG or CpNG in their promoter regions. When genes with enhanced expression in a particular phenotype are selected as target "genes" for expression suppression, their expression is suppressed by methylating the gene promoters using the above-described gene-suppressing agents. Thus, by selecting genes involved in a particular phenotype, the gene-suppressing agents of the present invention are useful as reagents for analyzing that particular phenotype, or as tools for preparing knockdown animals, and thus are extremely useful in biochemistry.

When selecting genes with enhanced expression in diseases as the above-described "genes", the above-described gene expression-suppressing agents are used to suppress their expression by methylation of the gene promoters involved in the diseases. In fact, by selecting a gene associated with tumor genesis, for example, erbB, and then introducing cancer cells with dsRNAs targeted to CpG islands in the erbB promoter, the proliferation of cancer cells can be suppressed by suppressing the expression of erbB. Thus, the gene expression-suppressing agents targeted to cancer-associated genes can be used as reagents for studying diseases such as cancer, or therapeutic agents for cancers in the medical field.

### Brief Description of the Drawings

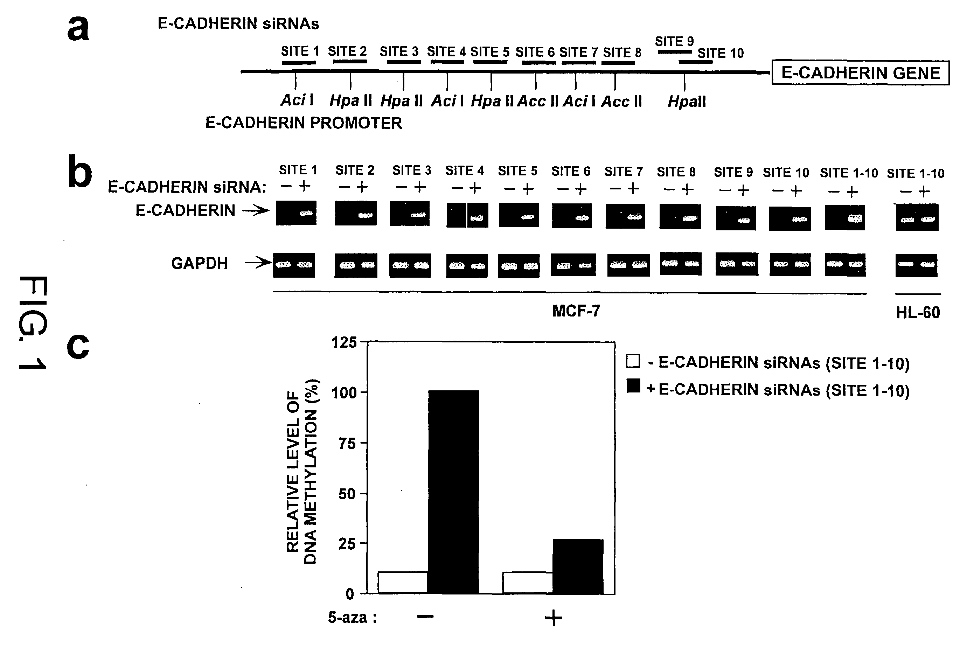

Fig. 1 is diagrams and photographs showing the siRNA-directed DNA methylation of E-cadherin promoter. a) A diagram showing siRNA target sites in the E-cadherin promoter. Each of the sites (1 to 10) contains one or two CpG sequences. *Hpa*I, *Aci*I, *Acc*I, and *Hha*I are CpG methylation-sensitive restriction enzymes. b) Photographs showing the detection analysis of siRNA-directed DNA methylation of E-cadherin promoter using a PCR-based methylation assay method. All siRNAs targeted to CpG sites induced DNA methylation in the promoter. GAPDH is a control gene that is resistant to *Hpa*I, *Aci*I, *Acc*I, and *Hha*I restriction digestion. It is obvious that the levels of amplified GAPDH gene fragment are nearly the same in all samples, and fall within the range of experimental error. c) A diagram showing the relative degree of DNA methylation in the E-cadherin promoter in the presence or absence of methylation inhibitor 5-aza. The siRNA-directed DNA methylation of the E-cadherin promoter was detected by a PCR-based methylation assay method. Band intensity was determined by densitometry using the NIH Image analysis program.

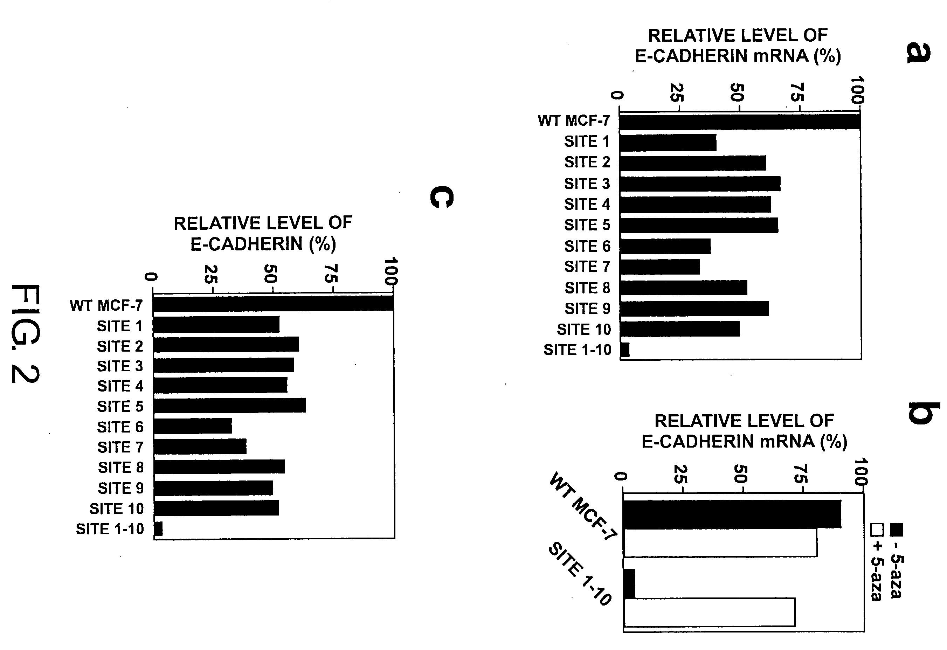

Fig. 2 is diagrams showing the effect of E-cadherin-siRNA targeted to CpG sites of E-cadherin promoter. a) A graph showing the relative level of E-cadherin mRNA in cells comprising E-cadherin-siRNA. Total RNA in each cell sample was analyzed by RT-PCR. The level of E-cadherin mRNA was normalized using the level of GAPDH mRNA. b) The relative level of E-cadherin mRNA in the presence or absence of 5-aza in cells comprising E-cadherin-siRNA. Total RNA in each cell sample was analyzed by RT-PCR. c) The relative level of E-cadherin in cells comprising E-cadherin-siRNA. E-cadherin in each of the cell samples was detected by Western blotting using specific antibodies. The relative levels of E-cadherin in each of the cell samples were determined by densitometry using the NIH Image analysis program.

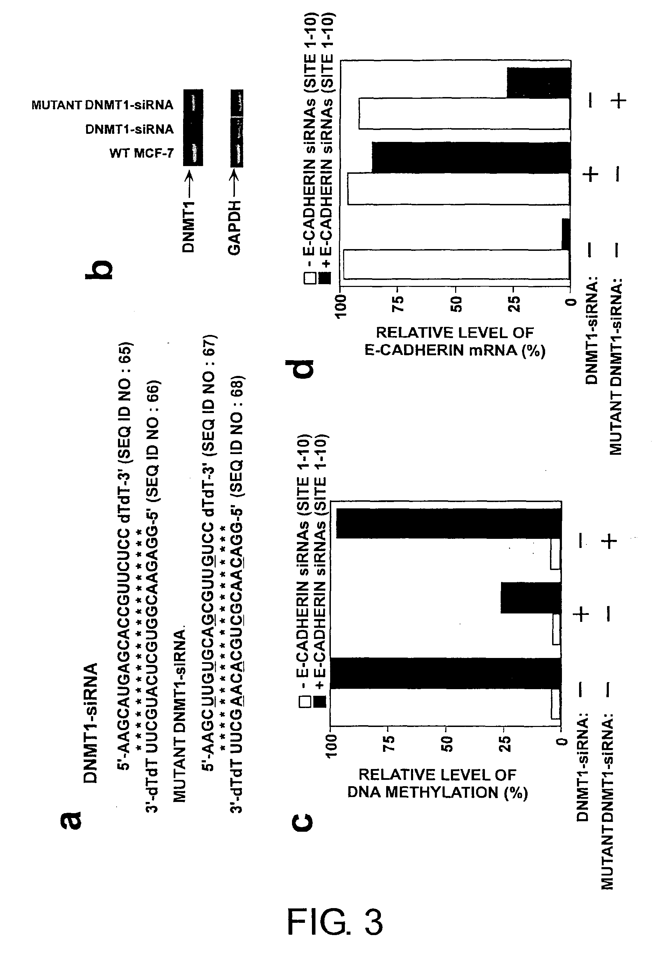

Fig. 3 is diagrams and photographs showing the effect of E-cadherin-siRNA that is targeted to E-cadherin promoter in DNMT1-knockdown cells. a) The sequences of DNMT1-siRNA and mutant DNMT1-siRNA. Underlined letters represent nucleotide mutations in DNMT-1-siRNA. b) Photographs showing the level of DNMT-1 mRNA in the presence or absence of DNMT1-siRNA in MCF-7 cells. The level of DNMT-1 mRNA was determined by RT-PCR. c) A graph showing the degree of DNA methylation in the E-cadherin promoter in the presence or absence of E-cadherin-siRNA and DNMT-1-siRNA in MCF-7 cells. The siRNA-directed DNA methylation in the E-cadherin promoter was detected by a PCR-based methylation assay method. Band intensity was determined by densitometry using the NIH Image analysis program. d) A graph showing the level of E-cadherin mRNA in the presence or absence of E-cadherin-siRNA and DNMT1-siRNA in MCF-7 cells. Total RNA in each cell sample was analyzed by RT-PCR using specific primers.

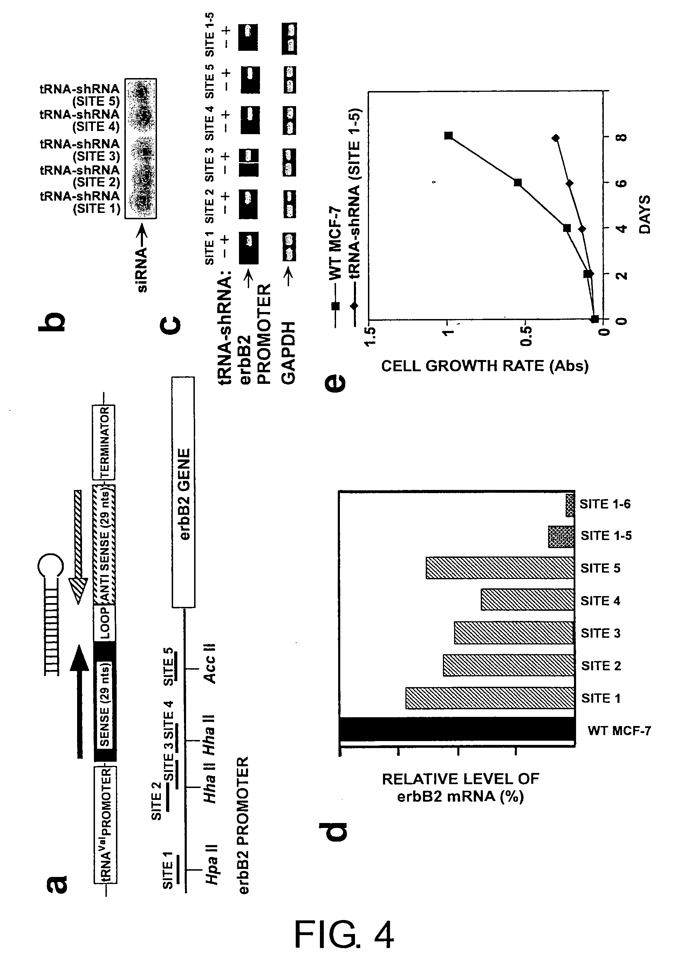

Fig. 4 is diagrams and photographs showing tRNA-shRNA-directed DNA methylation in erbB2 promoter. a) A diagram showing the structure of a tRNA-shRNA vector targeted to erbB2 promoter. Five CpG sites in the erbB2 promoter were selected as targets for tRNA-shRNAs. b) A photograph showing the detection results for siRNAs expressed from tRNA-shRNAs by Northern blotting. All siRNAs were detected in cells expressing tRNA-shRNAs in the promoter erbB2. c) Photographs showing the results of inducing tRNA-shRNA-directed DNA methylation in the erbB2 promoter. The siRNA-directed DNA methylation of erbB2 promoter was detected by a PCR-based methylation assay method. Band intensity was determined by densitometry using the NIH Image analysis program. d) A graph showing the relative level of erbB2 mRNA in cells expressing tRNA-shRNA targeted to the erbB2 promoter (sites 1 to 5), and the relative level of erbB2 mRNA in cells also expressing tRNA-shRNA targeted to the coding region (site 6). Total RNA from each of the cell samples was analyzed by RT-PCR using specific primers. e) A graph showing the growth rate of cells expressing tRNA-dsRNAs. The growth rate was determined by procedures as described in the methods section.

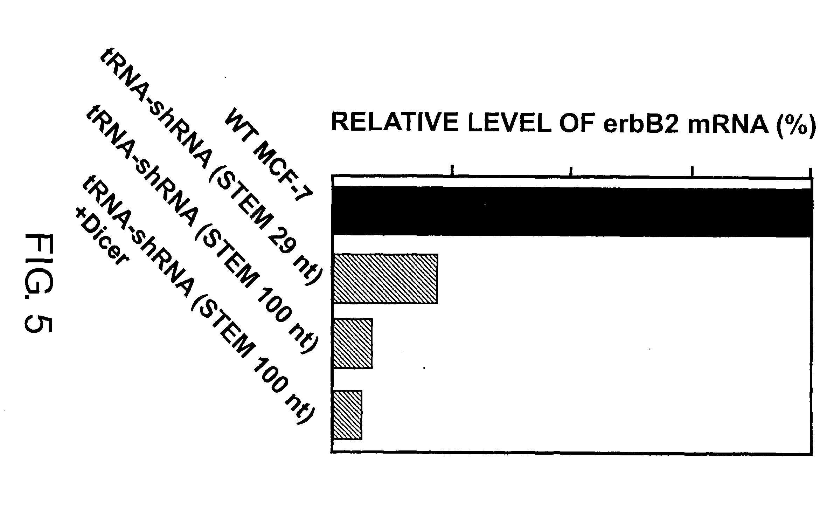

Fig. 5 is a graph showing the relative level of erbB2 mRNA in MCF-7 cells into which slightly longer shRNA (with stem lengths ranging from 29 to 100 nt) targeted to the erbB2 promoter was introduced alone, or in combination with tRNA-shRNA encoding Dicer. Total RNA from each of the cell samples was analyzed by RT-PCR using specific primers.

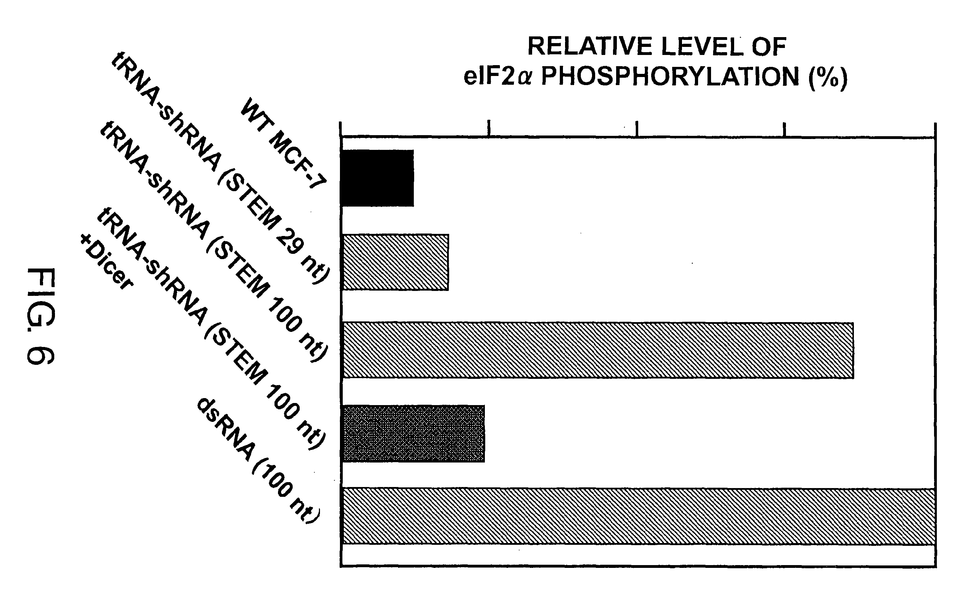

Fig. 6 is a graph showing the analysis results for the presence or absence of PKR activation in MCF-7 cells into which long hRNA was introduced alone, or in combination with tRNA-shRNA vector or the like capable of expressing Dicer. The activation was analyzed based on the degree of eIF2α phosphorylation.

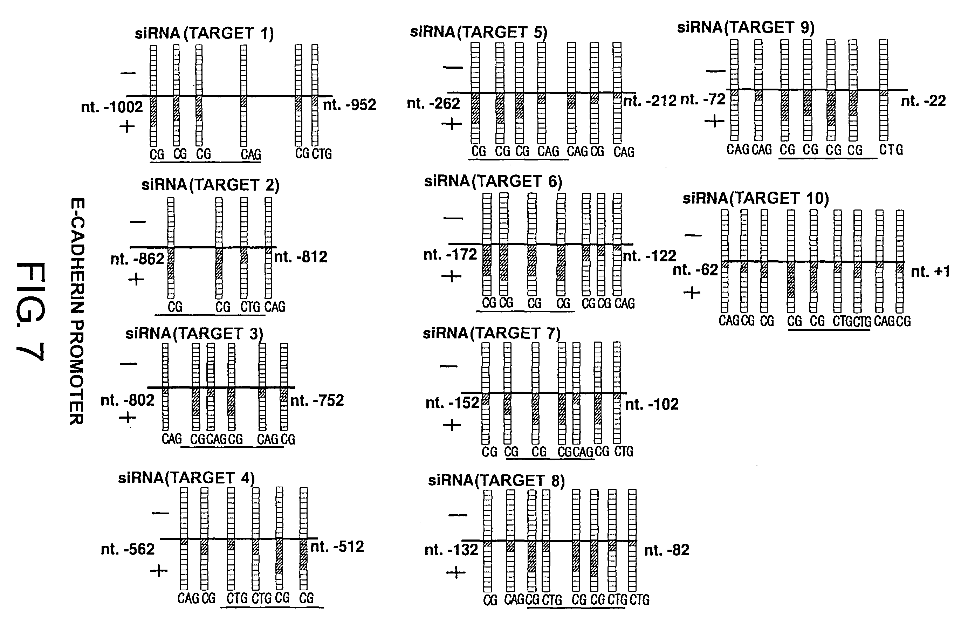

Fig. 7 is a diagram showing detection results for sequence-specific methylation in MCF-7 cells to which siRNAs targeted to the E-cadherin promoter were introduced. Shaded boxes indicate that corresponding sequences in site 1 or 3 were methylated when siRNA against site 1 or site 3 had been introduced (+) or not introduced (-) into the cells. Not only CpG sequences but also CpNG sequences were found to serve as target sequences for methylation. A similar result was obtained for the comparison between the presence and absence of siRNA targeted to site 3. Sequence-specific methylation was detected by bisulfite sequencing.

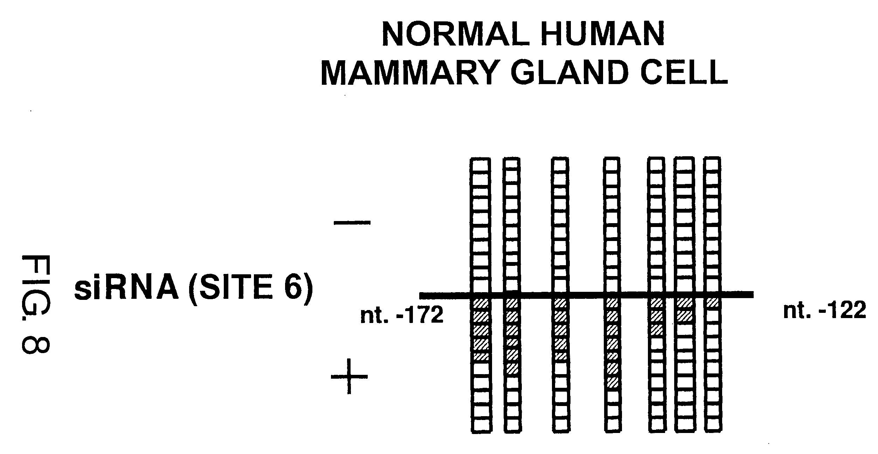

Fig. 8 is a diagram showing detection results indicating that siRNA-directed methylation was also induced in normal human mammary gland cells. Sequence-specific methylation was detected by bisulfite sequencing.

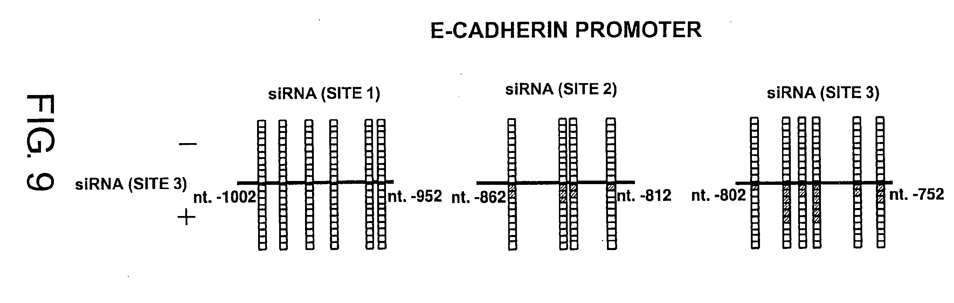

Fig. 9 is a diagram showing the range of methylated regions in E-cadherin promoter in MCF-7 cells to which siRNAs targeted to the E-cadherin promoter is introduced. The results indicate that CpG sequences adjacent to the target region were also methylated. The siRNA for site 3 also methylated the site 2 region. The sequence-specific methylation was detected by the bisulfite sequencing method described above.

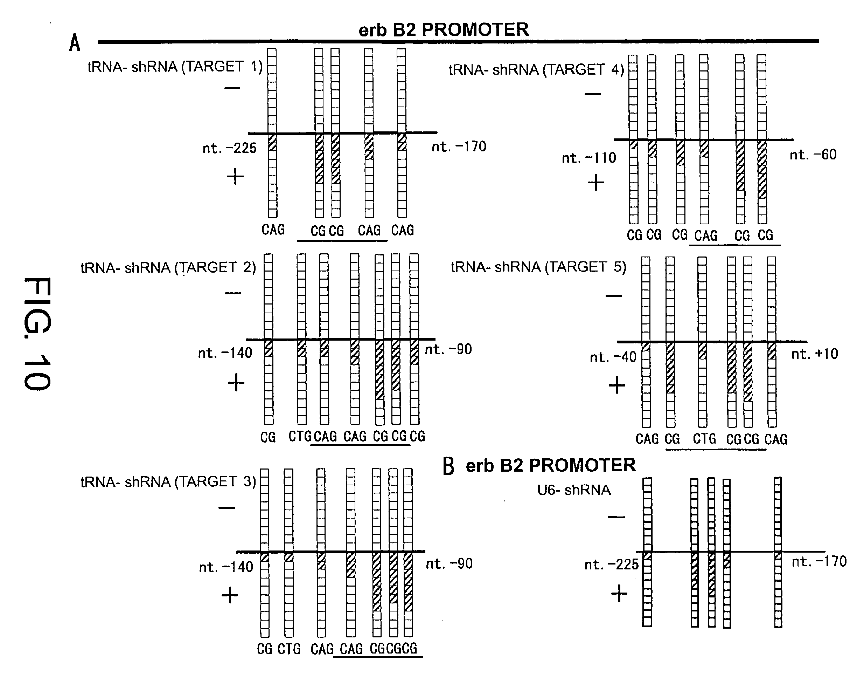

Fig. 10 is a diagram showing the results of inducing sequence-specific methylation using expression vectors for shRNA (tRNA-shRNA and U6-shRNA) targeted to the erbB2 promoter.

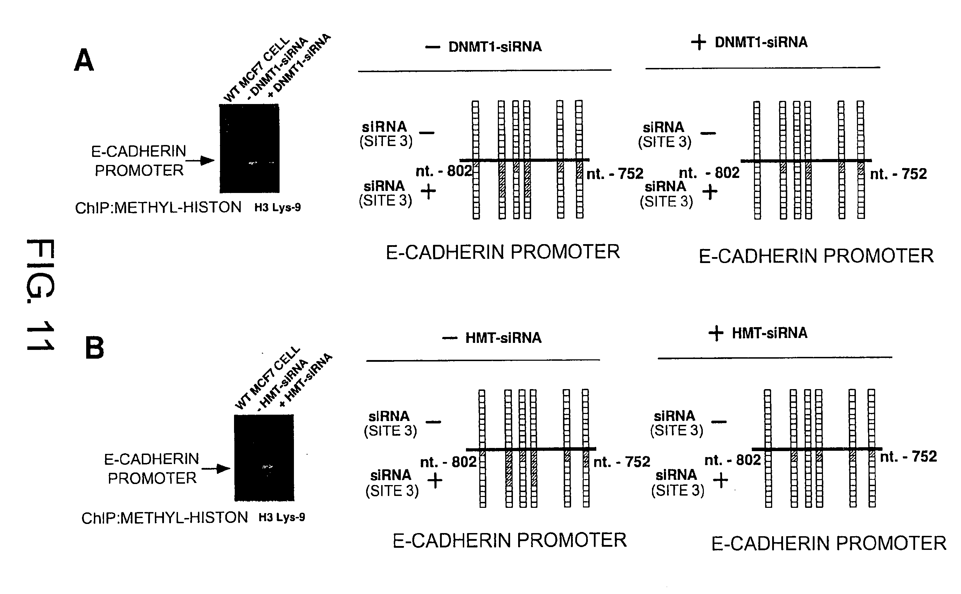

Fig. 11 is diagrams showing the analysis results for the influence of suppression of expression of methyl transferase DNMT1 or HMT by siRNA on the methylation induced by siRNA targeted to E-cadherin promoter. A) Analysis of differences in the degree of sequence-specific methylation when using siRNA targeted to the E-cadherin promoter in the presence or absence of siRNA targeted to DNMT1 promoter. B) Analysis of differences in the degree of sequence-specific methylation when using siRNA targeted to the E-cadherin promoter in the presence or absence of siRNA targeted to HMT promoter. Sequence-specific methylation was detected by the bisulfite sequencing method as described above.
Fig. 12 is a diagram showing the construction of the dsRNA expression vector (pSV-BGI), which was designed to allow long dsRNA to localize in the nucleus. The vector comprises a PolII promoter. The gene encoding long dsRNAs is inserted into an intron of the beta globulin gene, whose expression is driven by the promoter. Introns are processed in the nucleus. Therefore, the long dsRNAs inserted into the intron are released in the nucleus.

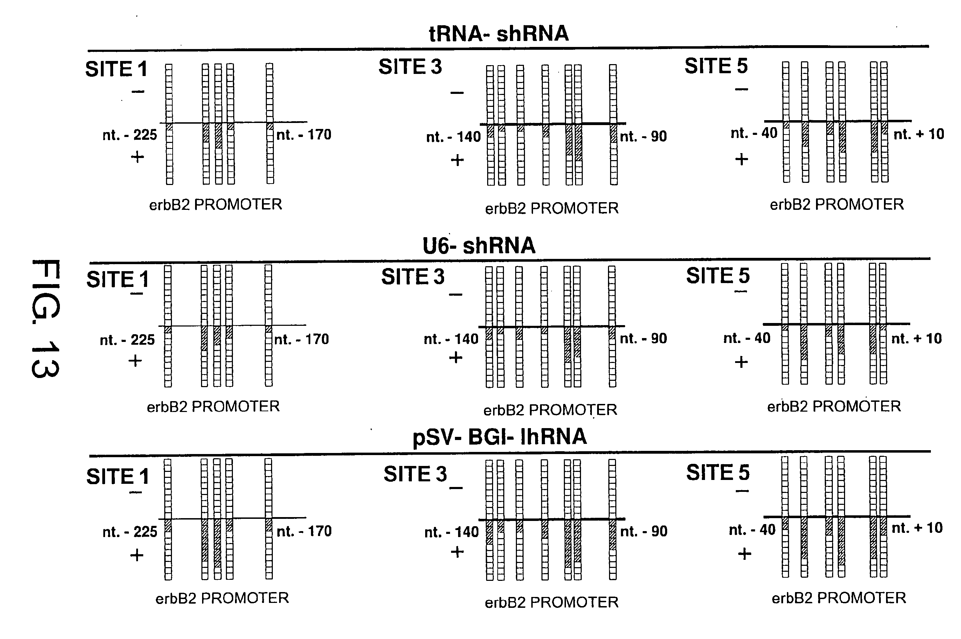

Fig. 13 is a diagram showing the results of inducing methylation in the erbB2 promoter using a long dsRNA expression vector carrying tRNA promoter or U6 promoter, or the expression vector pSV-BGI for long dsRNA in the nucleus.

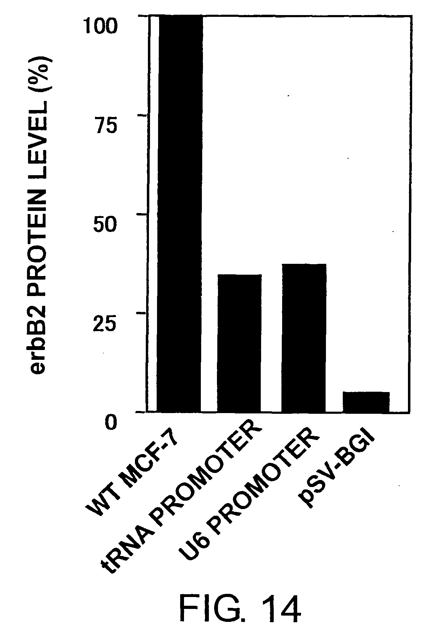

Fig. 14 is a graph comparing the expression level of erbB2 protein in MCF-7 cells to which a long dsRNA expression vector carrying a tRNA promoter or U6 promoter, or the expression vector pSV-BGI for long dsRNA in the nucleus is introduced.

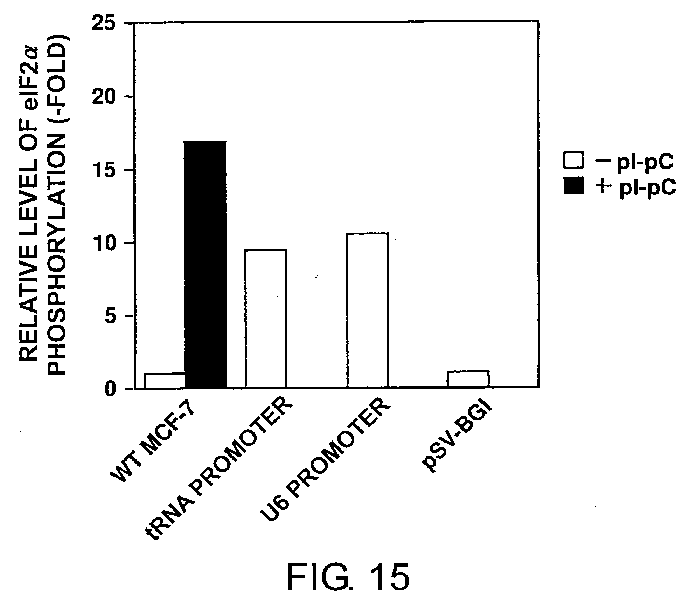

Fig. 15 is a graph showing the cytotoxicity of each vector expressing long dsRNA. Cytotoxicity was assessed using, as an indicator, PKR activation in cells to which each vector is introduced. PKR activation was tested by determining the degree of eIF2α phosphorylation.

### Best Mode for Carrying Out the Invention

Herein below, the present invention will be specifically described using Examples, however, it is not to be construed as being limited thereto.

The "siRNAs" refer solely to short dsRNAs in all of the Examples described below, and short dsRNAs, such as for the purpose of RNA interference, were used to induce DNA methylation. Thus, unless otherwise specified, herein, siRNAs refer to short dsRNAs used to induce DNA methylation.

### Example 1: Synthetic siRNA-directed DNA methylation in mammalian cells

The present inventors synthesized siRNA targeted to CpG islands of E-cadherin promoter (E-cadherin-siRNA) to test whether synthetic siRNAs can induce DNA methylation in mammalian cells.

It has been reported that in some tumor cell lines E-cadherin is silenced by aberrant methylation of its promoter (Herman, J.G. *et al*. Proc. Natl. Acad. Sci. USA. 93, 9821-9826 (1996); Graff, J.R. *et al*. J. Biol. Chem. 272, 22322-22329 (1997); Com, P.G. *et al*. Clin. Cancer Res. 6, 4243-4248 (2000)). The present inventors used MCF-7 cells in the present study because CpG sites in the E-cadherin promoter in MCF-7 cells are not methylated. As a control the inventors used HL-60 cells, whose E-cadherin promoter CpG sites are methylated (Corn, P.G. *et al.* Clin. Cancer Res. 6, 4243-4248 (2000)). Ten CpG sites in the E-cadherin promoter (sites 1 to 10) were used as siRNA targets (Fig. 1a).

Japan Bio Services Co., Ltd synthesized the siRNAs targeting the E-cadherin promoter (E-cadherin-siRNA), and the siRNAs targeting DMNT1 mRNA (DMNT1-siRNA) (JbioS; Saitama, Japan). The target sequences for E-cadherin-siRNA are as follows:

Site 1 (SEQ ID NO: 1) 5'-AGG CCG CUC GAG CGA GAG UGC AGU G-3'

Site 2 (SEQ ID NO: 2) 5'-GCC GGU GUG GUG GCA CAC GCC UGU A-3'

Site 3 (SEQ ID NO: 3) 5'-CCG GCA GGC GGA GGU UGC AGU GAG C-3'

Site 4 (SEQ ID NO: 4) 5'-ACU GCC CCU GUC CGC CCC GAC UUG U-3'

Site 5 (SEQ ID NO: 5) 5'-CGG CGG GGC UGG GAU UCG AAC CCA G-3'

Site 6 (SEQ ID NO: 6) 5'-UCA CCG CGU CUA UGC GAG GCC GGG U-3'

Site 7 (SEQ ID NO: 7) 5'-CGG GUG GGC GGG CCG UCA GCU CCG C-3'

Site 8 (SEQ ID NO: 8) 5'-CGC CCU GGG GAG GGG UCC GCG CUG C-3'

Site 9 (SEQ ID NO: 9) 5'-GCG GUA CGG GGG GCG GUG CUC CGG G-3'

Site 10 (SEQ ID NO: 10) 5'-CGG UGC UCC GGG GCU CAC CUG GCU G-3'

Then, to prepare siRNAs of 25 nucleotides against these target sites, the respective synthetic sense and antisense RNA strands were annealed by a conventional method (Elbashir, S.M. *et al*. Genes Dev. 15, 188-200 (2001)).

MCF-7 cells were cultured in Dulbecco's modified Eagle medium (DMEM) containing 10% fetal bovine serum (FBS). Each of the above E-cadherin-siRNAs (200 nM) was introduced into the MCF-7 cells using Oligofectamine™ (Invitrogen, CA, USA) according the supplier's protocol. After introducing the siRNAs, the cells were cultured for 96 hours. Then, total DNAs were collected from the cells, and genomic DNAs were isolated from the DNAs.

PCR-based methylation analysis was then carried out. Specifically, the isolated genomic DNAs described above were digested with methylation-sensitive enzymes (*Hpa*I, *Aci*I, *Acc*I, and *Hha*I; Fig. 1a) under conditions recommended in the supplier's protocol. After enzymatic digestion, the E-cadherin promoter region was amplified by PCR from 50 ng of DNA using the promoters as listed below: E-cadherin forward primer ((SEQ ID NO: 29) 5'-TCT AGA AAA ATT TTT TAA AA-3') and reverse primer ((SEQ ID NO: 30) 5'-AGA GGG GGT GCG TGG CTG CA-3'); or erbB2 forward primer ((SEQ ID NO: 31) 5'-CCC GGG GGT CCT GGAAGC CA-3') and reverse primer ((SEQ ID NO: 32) 5'-CCC GGG GGG GCT CCC TGG TTT-3').

This analysis is based on the principle that the methylation-sensitive enzymes described above cleave un-methylated CpG islands in promoters, and thus the DNAs are not amplified (Qian, X. *et al*. Am. J. Pathol. 153, 1475-1482 (1998)). Based on this principle, the methylation of CpG islands in promoters can be detected by detecting the amplified products of this analysis.

In a control experiment, intact genomic DNA of E-cadherin promoter was treated with *Pst*I, which is capable of cleaving E-cadherin promoter, and *Hind*III, which is incapable of cleaving the promoter. All samples were digested by each of the enzymes in two independent experiments, to exclude the possibility of incomplete digestion. PCR amplification was repeated at least twice for each of the two lots of digestion products. To prevent over-cycling in the PCR reaction, the cycle curve number method was used to determine the number of cycles required for each primer set, using an intact template and the template digested with restriction enzyme *Pst*I (which gives no PCR products).

Primers specific to E-cadherin are listed below.

Site 1:
forward primer ((SEQ ID NO: 33) 5'-TCT AGA AAA ATT TTT TAA AAA A-3') and
reverse primer ((SEQ ID NO: 34) 5'-AGC CTC CTG AAG TGT TGG ATT A-3')

Site 2:
forward primer ((SEQ ID NO: 35) 5'-ACA TGG TGA AAC CCC GTC TTG T-3') and
reverse primer ((SEQ ID NO: 36) 5'-TGT CTC AGC CTA TTG AGT AGC T-3')

Site 3:
forward primer ((SEQ ID NO: 37) 5'-TAG GCT GAG ACA GGA GAG TCT C-3') and
reverse primer ((SEQ ID NO: 38) 5'-CCA GGC TGG AGT GCA GTG GCA C-3')

Site 4:

forward primer ((SEQ ID NO: 39) 5'-GGC AAT ACA GGG AGA CAC AGC G-3') and
reverse primer ((SEQ ID NO: 40) 5'-ACA CCA CCA CGC CAG GCT AAT T-3')

Site 5:
forward primer ((SEQ ID NO: 41) 5'-TTC TGA TCC CAG GTC TTA GTG A-3') and
reverse primer ((SEQ ID NO: 42) 5'-TAG GTG GGT TAT GGG ACC TGC A-3')

Site 6:
forward primer ((SEQ ID NO: 43) 5'-AGC AAC TCC AGG CTA GAG GGT C-3') and
reverse primer ((SEQ ID NO: 44) 5'-GCG CGG ACC CCT CCC CAG GGC G-3')

Site 7:
forward primer ((SEQ ID NO: 45) 5'-GGC TAG AGG GTC ACC GCG TCT A-3') and
reverse primer ((SEQ ID NO: 46) 5'-TAC CGC TGA TTG GCT GAG GG-3')

Site 8:
forward primer ((SEQ ID NO: 47) 5'-CGG GTG GGC GGG CCG TCA GCT-3') and
reverse primer ((SEQ ID NO: 48) 5'-ATT GGC TGA GGG TTC ACC TGC C-3')

Site 9:
forward primer ((SEQ ID NO: 49) 5'-GGC AGG TGA ACC CTC AGC CAA T-3') and
reverse primer ((SEQ ID NO: 50) 5'-TGC GTG GCT GCA GCC AGG TGA G-3')

Site 10:
forward primer ((SEQ ID NO: 51) 5'-GGC AGG TGA ACC CTC AGC CAA T-3') and
reverse primer ((SEQ ID NO: 52) 5'-TGC GTG GCT GCA GCC AGG TGA G-3')

GAPDH was used as a control gene. After enzymatic treatment with *Hpa*I, *Aci*I, *Acc*I, *Hha*I, *Pst*I, and *Hind*III, which are incapable of digesting the GAPDH gene, DNA fragments were amplified by PCR using the specific forward primer ((SEQ ID NO: 63) 5'-GTC TTC ACC ACC ATG GAG AAG GCT-3') and reverse primer ((SEQ ID NO: 64) 5'-GCC ATC CAC AGT CTT CTG GGT GGC-3'). The amplification levels of partial GAPDH gene in the respective samples were thus found to be comparable, and to fall within the limits of experimental error.

As shown in Fig. 1b, methylated DNAs were detected in MCF-7 cell lines each comprising individual E-cadherin-siRNA (sites 1 to 10) or a mixture of all E-cadherin-siRNAs. Furthermore, in MCF-7 cells containing the mixture of all E-cadherin-siRNAs, the level of methylated DNA was reduced in the presence of DNA methylation inhibitor 5-aza-2'-deoxycytidine (abbreviated to 5-aza), (Fig. 1c). These findings suggest that siRNA targeted to the CpG region in the E-cadherin promoter can induce sequence-specific DNA methylation.

### Example 2: Correlation of DNA methylation directed by E-cadherin-siRNA and expression of the E-cadherin gene

The present inventors carried out RT-PCR using primers specific to the E-cadherin promoter to test whether E-cadherin-siRNA-directed DNA methylation had a correlation with expression of the E-cadherin gene.

Total RNAs were isolated from MGF-7 cells using ISOGEN™ (Nippon Gene; Toyama, Japan) according the supplier's protocol. RT-PCR was carried out using RNA PCR Kit ver. 2 (TaKaRa) and the following primers:

E-cadherin forward primer ((SEQ ID NO: 21) 5'-ATG GGC CCT TGG AGC CGC AGC
CTC-3') and downstream primer ((SEQ ID NO: 22) 5'-GAG CAA TTC TGC TTG GAT TCC
AGA-3')

Control GAPDH forward primer ((SEQ ID NO: 27) 5'-ATG GGG AAG GTG AAG GTC
GGA GTC-3') and reverse primer ((SEQ ID NO: 28) 5'-TGG AAT TTG CCA TGG GTG
GA-3')

The PCR products amplified using the above primers were analyzed by electrophoresis in a 2% agarose gel.

As shown in Fig. 2a, the levels of E-cadherin mRNA in MCF-7 cell lines each comprising individual E-cadherin-siRNAs were approximately 30% to 70% lower than that in the wild-type (WT) MCF-7 cell.

In contrast, the E-cadherin mRNA level in MCF-7 cells containing a mixture of all E-cadherin-siRNAs was significantly lower than those in MCF-7 cells to which any one of the siRNAs had been transfected. This suggests the existence of an additive effect (the "sites 1 to 10" columns in the Figure). It is noteworthy that treatment with 5-aza-2'-dC almost completely stopped the activities of E-cadherin-siRNAs (Fig. 2b).

The level of E-cadherin protein was determined by Western blotting analysis using an antibody specific to E-cadherin.

Western blotting analysis was carried out by previously reported methods (Kawasaki, H. *et al*. Nature, 393, 284-289 (1998); Kawasaki, H. *et al*. Nature 405, 195-200 (2000)). MCF-7 cells comprising each tRNA-shRNA targeted to each of the E-cadherin-siRNAs targeting the E-cadherin promoter were individually harvested. Total proteins (20 µg each) were separated by SDS-PAGE (10% polyacrylamide gel) and then transferred to a polyvinylene difluoride (PVDF) membrane (Funakoshi; Tokyo, Japan) by electroblotting. The resulting immune complexes were visualized with polyclonal antibodies specific to E-cadherin (Santa Cruz; CA, USA) and to actin (UBI; CA, USA) using an ECL kit (Amersham Co.; Buckinghamshire, UK). The relative levels of E-cadherin were normalized based on the actin levels.

As shown in Fig. 2c, Western blotting analysis using antibody specific to E-cadherin, as described above, was used to confirm that E-cadherin was reduced not only at the mRNA level, but also at the protein level. These findings clearly indicate that siRNAs targeted to the E-cadherin promoter served as gene silencers at the transcriptional level. In addition, it was found that siRNA-directed DNA methylation in the E-cadherin gene promoter was reverse-correlated with gene expression level in an additive fashion.

### Example 3: Involvement of DNMT in siRNA-directed DNA methylation in human cells

It is known that DNA methylation in mammalian cells is typically caused by DNA methyltransferase (DNMT) (Bestor, T.H. Human Mol. Genet. 9, 2395-2402 (2000)). The present inventors tried to suppress DNMT gene expression using siRNAs targeted to DMNT mRNA, to examine whether DNMT is involved in siRNA-directed DNA methylation in human cells. DNMT1 is a major DNA methyltransferases in human cells (Rhee, I. *et al.* Nature 416, 552-556 (2002); Robert, M. F. *et al.* Nature Genet. 33, 61-65 (2003)). Thus, the present inventors synthesized siRNA targeted to DNMT1 mRNA (Robert, M. F. *et al.* Nature Genet. 33, 61-65 (2003)) (Fig. 3a). A mutant DNMT1-siRNA in which the sense and antisense strands each comprise four point mutations was used as a control.

The sequences of DMNT1-siRNA and mutant DMNT1-siRNA are shown in Fig. 3a (SEQ ID NOs: 65 to 68). To prepare these siRNAs, the sense and antisense synthetic RNA strands were respectively annealed by a conventional method (Elbashir, S.M. *et al.* Genes Dev. 15, 188-200 (2001)).

The above-described DNMT1-siRNA and mutant DNMT1-siRNA (200 nM each) were each introduced into MCF-7 cells using Oligofectamine™ by the same procedure as described in Example 2. 96 hours after introduction, total RNAs were collected using ISOGEN™ (Nippon Gene; Toyama, Japan). The levels of DNMT1 mRNA were determined by RT-PCR.

RT-PCR was carried out using RNA PCR Kit ver. 2 (TaKaRa) and the following primers: DNMT1 forward primer ((SEQ ID NO: 25) 5'-ATG GCT CGC GCC AAAACA GTC ATG A-3') and reverse primer ((SEQ ID NO: 26) 5'-CTC GGG ACT GGG ATC CAT GAG AAT-3'). Control experiments were carried out using GAPDH forward primer ((SEQ ID NO: 27) 5'-ATG GGG AAG GTG AAG GTC GGA GTC-3') and reverse primer ((SEQ ID NO: 28) 5'-TGG AAT TTG CCA TGG GTG GA-3') by the same procedure. The resulting PCR products were analyzed by electrophoresis in a 2% agarose gel.

As shown in Fig. 3b, the level of DNMT1 mRNA in MCF-7 cells containing DNMT1-siRNA was significantly lower than that in WT-MCF-7 cells. The DNMT1 mRNA level in MCF-7 cells containing mutant DNMT1-siRNA was found to be comparable to that in wild-type MCF-7 cells.

The level of DNMT-1 protein in the MCF-7 cells to which DNMT1-siRNA was introduced was also determined by Western blotting analysis using a DNMT1-specific antibody. The resulting data is not shown in the drawings herein. The introduction of DNMT1-siRNA was confirmed to decrease DNMT-1 not only at the mRNA level, but also at the protein level.

Then, a mixture of the above E-cadherin-siRNAs (sites 1 to 10) was introduced into MCF-7 cells containing DNMT1-siRNA to evaluate the influence of the reduced level of DNMT-1 gene expression on siRNA-directed DNA methylation. The siRNAs were introduced using Oligofectamine™. After 96 hours, the total DNAs were collected from the cells, and genomic DNAs were isolated from the DNAs. The degree of methylation in the E-cadherin promoter region was analyzed using the isolated genomic DNAs and the PCR-based analytical method for methylation described in Example 1.

As shown in Fig. 3c, the degree of DNA methylation directed by E-cadherin-siRNA in MCF-7 cells containing DNMT1-siRNA was significantly lower than that in WT MCF-7 cells and in MCF-7 cells containing the mutant DNMT1-siRNA. Thus, it was clarified that DNMT1 was essential for siRNA-directed DNA methylation in human cells.

Furthermore, whether E-cadherin-siRNA influenced the expression of E-cadherin in MCF-7 cells containing DNMT1-siRNA was also examined. Specifically, DNMT1-siRNA (or the mutant DNMT1-siRNA) was introduced into MCF-7 cells, and then E-cadherin-siRNA was further introduced into the same cells. These siRNAs were introduced using the kit described in Example 1. 96 hours after introduction of E-cadherin-siRNA, total RNAs were collected from the MCF-7 cells and the levels of E-cadherin mRNA were determined by RT-PCR. The RT-PCR was carried out by the procedure described in Example 2.

As shown in Fig. 3d, siRNA targeted to E-cadherin promoter did not alter the expression level of E-cadherin gene in MCF-7 cells containing siRNAs (DNMT1-siRNA) targeted to DNMT1 mRNA. In contrast, E-cadherin-siRNA decreased the level of E-cadherin gene expression in MCF-7 cells containing mutant DNMT1-siRNA. These findings suggest that the inhibition of DNA methylation by DNMT1-siRNA influences E-cadherin-siRNA-mediated gene silencing at a transcriptional level.

### Example 4: Suppression of erbB2 gene expression using an expression vector for hairpin RNA (shRNA)

The erbB2 gene is known to be over-expressed and also unmethylated in some tumor cell lines such as MCF-7 cell line. Meanwhile, the erbB2 gene is silenced upon methylation of its promoter in various normal cell lines (Hattori, M. *et al*. Cancer Lett. 169, 155-164 (2001)). The present inventors constructed expression vectors for short hairpin RNA (shRNA) targeted to the above erbB2 promoter, taking into consideration future gene therapy by regulating DNA methylation using siRNA.

The present inventors have previously demonstrated that shRNA generated by tRNA^{val} induces siRNA-directed gene silencing in human cells (Kawasaki, H., & Taira, K. Nucleic Acids Res. 31, 700-707 (2003)). Hence, the inventors constructed an expression vector for tRNA-shRNA targeted to the erbB2 promoter using pPUR-tRNA plasmid which comprises a pPUR (Clontech, CA, USA) backbone and a synthetic human gene promoter for tRNA^{val} inserted between the *Eco*RI and *Bam*HI sites (Kawasaki, H., & Taira, K. Nucleic Acids Res. 31, 700-707 (2003)). Five CpU islands (sites 1 to 5) in the erbB2 promoter were selected as the target sequences for tRNA-shRNA targeted to the erbB2 promoter. The target sequences (sites 1 to 5) are shown below.

Site 1 (SEQ ID NO: 11) 5'-UUA UCC CGG ACU CCG GGG GAG GGG GCA GAG-3'

Site 2 (SEQ ID NO: 12) 5'-UGC AGG CAA CCC AGC UUC CCG GCG CUA GGA-3'

Site 3 (SEQ ID NO: 13) 5'-CCA GCU UCC CGG CGC UAG GAG GGA CGC ACC-3'

Site 4 (SEQ ID NO: 14) 5'-CAG GCC UGC GCG AAG AGA GGG AGA AAG UGA-3'

Site 5 (SEQ ID NO: 15) 5'-GGA GGG GGC GAG CUG GGA GCG CGC UUG CUC-3'

A synthetic oligonucleotide encoding dsRNA, which is targeted to the above-described erbB2 promoter and comprises a loop motif, was prepared as a double-stranded sequence by PCR. After digestion with *Sac*I and *Kpn*I, the resulting fragment was cloned into the above-described pPUR-tRNA, downstream of the tRNA gene promoter.

Then, whether shRNAs of interest were generated from the expression plasmids for shRNA in MCF-7 cells was confirmed experimentally. The shRNA expression plasmids were introduced transiently into MCF-7 cells by the same method described in Example 1. 72 hours after introduction, total RNAs were extracted and analyzed by Northern blotting. The results showed that proper processing had occurred to generate shRNA in cells expressing tRNA-shRNA (Fig. 4b). After confirming the generation in cells of shRNA by using the expression vectors, these expression vectors were used to induce methylation of the erbB2 promoter.

It was then tested whether the degree of DNA methylation in the erbB2 promoter was altered by the shRNA generated using each of the expression vectors as described above. This analysis was carried out by the PCR-based method for analyzing methylation, as described in Example 2. Specifically, each pPUR-tRNA-shRNA expression vector was introduced into MCF-7 cells using Effectin™ reagent (QIAGEN, Hiddeln, Germany) according to the attached supplier's protocol. 96 hours after introduction, genomic DNAs were collected from the cells, and the degree of methylation in the erbB2 promoter was determined.

The genomic DNA (500 ng) was digested with the methylation-sensitive enzymes, *Hpa*I and *Hha*I (Fig. 4a). After digestion, 50 ng of DNA was amplified by PCR using following promoters: erbB2 forward primer ((SEQ ID NO: 31) 5'-CCC GGG GGT CCT GGA AGC CA-3') and reverse primer ((SEQ ID NO: 32) 5'-CCC GGG GGG GCT CCC TGG TTT-3'). In a control experiment, intact genomic DNA for the erbB2 promoter was treated with *Pst*I, which is capable of cleaving the erbB2 promoter, and *Hind*III, which is incapable of cleaving the promoter.

As in Example 1, all samples were digested by each of the enzymes in two independent experiments, to exclude the possibility of incomplete digestion. PCR amplification was repeated at least twice for each of the two lots of digestion products. To prevent over-cycling in the PCR reaction, the cycle curve number method was used to determine the number of cycles required for each primer set, using an intact template and the template digested with restriction enzyme *Pst*I (which gives no PCR products). Primers specific to earbB2 promoter are listed below:

Site 1:
forward primer ((SEQ ID NO: 53) 5'-CAG GAA AGT TTA AGA TAA AAC C-3') and
reverse primer ((SEQ ID NO: 54) 5'-CTC GGA GAA TCC CTA AAT GCA G-3')

Site 2:
forward primer ((SEQ ID NO: 55) 5'-CGA GGA AAA GTG TGA GAA CGG C-3') and
reverse primer ((SEQ ID NO: 56) 5'-CGC GCA GGC CTG GGT GCG TCC C-3')

Site 3:
forward primer ((SEQ ID NO: 57) 5'-CGA GGA AAA GTG TGA GAA CGG C-3') and
reverse primer ((SEQ ID NO: 58) 5'-CAG GCC TGG GTG CGT CCC TC-3')

Site 4:
forward primer ((SEQ ID NO: 59) 5'-GAG GGA CGC ACC CAG GCC TG-3') and
reverse primer ((SEQ ID NO: 60) 5'-CTG GGA GTG GCA ACT CCC AGC T-3')

Site 5:
forward primer ((SEQ ID NO: 61) 5'-AGA CTT GTT GGA ATG CAG TT-3') and
reverse primer ((SEQ ID NO: 62) 5'-CTT CAT TCT TAT ACT TCC TCA A-3')

GAIPDH was used as a control gene. After treatment with *Hpa*I, *Aci*I, *Acc*I, *Hha*I, *Pst*I, and *Hind*III, which are incapable of cleaving the gene, the fragment DNAs were amplified by PCR using a specific forward primer ((SEQ ID NO: 63) 5'-GTC TTC ACC ACC ATG GAG AAG GCT-3') and reverse primer ((SEQ ID NO: 64) 5'-GCC ATC CAC AGT CTT CTG GGT GGC-3'). The levels of amplified partial DAPDH gene in the respective samples were found to be comparable, and to fall within the limits of experimental error.

As shown in Fig. 4c, DNA methylation in the erbB2 promoter was detected without exception in MCF-7 cells expressing a tRNA-shRNA or a mixture of all tRNA-shRNAs (sites 1 to 5). A similar result was obtained with shRNAs generated using U6 promoter (data not shown). This indicates that vector-based siRNAs can also induce sequence-specific DNA methylation in human cells.

Then, the present inventors used RT-PCR to determine the level of erbB2 mRNA to test whether the vector-based siRNA suppressed erbB2 gene expression. RT-PCR was carried out by the same procedure as described in Example 2, except that an erbB2 forward primer ((SEQ ID NO: 23)5'-ATG GAG CTG GCG GCC TTG TGC CGC-3') and reverse primer ((SEQ ID NO: 24)5'-TTG TTC TTG TGG AAG ATG TCC TTC C-3') were used.

As shown in Fig. 4d, the levels of erbB2 mRNA in cells expressing individual tRNA-shRNA were lower in parts than that in WT MCF-7 cells. As shown above (Fig. 2a), the level of erbB2 mRNA in cells expressing all tRNA-shRNAs (1 to 5 sites) was significantly lower than that in WT MCF-7 cells or cells expressing any one of the tRNA-shRNAs. This indicates that vector-based shRNA suppression is additive at the transcriptional level. It thus suggests that transcriptional regulation based on siRNA-directed promoter methylation in mammalian cells can be a general mechanism, as shown by the present inventors using two examples of the present invention described herein.

A tRNA-shRNA targeted to the erbB2 gene-encoding region (site 6) was transfected in combination with all the above tRNA-shRNAs (1 to 5 sites) to MCF-7 cells and then RT-PCR was carried out by the same procedures as described above. As a result, the expression of the target gene was suppressed more efficiently, as shown in Fig. 4d.

Furthermore, the present inventors investigated the growth rates of various cell lines to assess the phenotypes of cells expressing tRNA-shRNA targeted to erbB2 gene promoter. The growth rate of each cell line was determined using a Cell Proliferation Kit II (Roche Ltd., Switzerland) according to the supplier's instructions (Kawasaki, H., & Taira, K. Nucleic Acids Res. 31, 700-707 (2003)).

As shown in Fig. 4e, MCF-7 cells expressing all tRNA-shRNAs (1 to 5 sites) proliferated significantly more slowly than wild-type MCF-7 cells. These results show that the decrease in the growth rate of MCF-7 cells expressing all tRNA-shRNAs (1 to 5 sites) is correlated with the decrease in the level of erbB2 mRNA in the cells. This suggests that the tRNA-shRNAs of the present inventors targeted to erbB2 gene promoter are useful as cell growth-suppressing agents, in particular as therapeutic agents to suppress neoplasms, such as cancer.

Sequencing is carried out to confirm the sequences of constructed expression vectors for the hairpin (stem-loop) RNA molecules described in this Example; however, since the stem-loop structures are rigid the sequences are difficult to analyze. Even when the structure of the stem-loop RNA molecules is changed to one for ease of sequence analysis, by introducing one to ten mismatch sequences or bulges into the sense strand of the stem sequences, the molecules still exhibit activity comparable to that of a perfectly complementary stem-loop RNA molecule with respect to DNA methylation or RNAi-mediated suppression of target genes (data not shown).

### Example 5: Co-expression system for long hRNAs and Dicer gene

The expression of long (30 nucleotides or more) hairpin (or stem-loop) RNA molecules ("long hRNAs") results in PKR activation and non-specific translation suppression in animal cells. Accordingly, to induce RNAi effect using hRNAs, stem-loop RNA molecules comprising 30 nucleotides or less must be prepared. Meanwhile, since the RNAi effect depends on the sequence of a target gene, many types of shRNAs should be prepared in order to select effective target sequences.

Thus, the present inventors devised a co-expression system for long hRNAs (25 to 500 nucleotides) and Dicer gene. Specifically, a vector was constructed to have both a Dicer expression cassette comprising Dicer gene linked downstream of a PolII promoter, and an hRNA expression cassette comprising short stem-loop RNA molecule encoding DNA linked downstream of a PolIII promoter, and this was then introduced into cells. Firstly, the expression-suppressing effect produced by the co-expression of Dicer gene and long hRNAs targeted to the coding region of erbB2 gene was found to be stronger than the effect obtained using conventional hRNAs (Fig. 5).

Furthermore, eIF2α phosphorylation was used as an indicator of PKR activation to analyze whether PKR was activated in this system. Specifically, Western blotting was carried out using an antibody which specifically recognizes phosphorylated eIF2α, and the amount of phosphorylated eIF2α relative to that of the internal control, β-actin, was estimated using the NIH Image program.

eIF2α phosphorylation was hardly observed in the results (Fig. 6). It was thus confirmed that PKR activation is hardly induced in this system.

This suggests that, in this system, Dicer was expressed and the co-expressed long stem-loop RNAs were rapidly processed to siRNAs in cells. Furthermore, PKR activation can be prevented more effectively by introducing mismatch sequences (G-T pairs and the like) and bulges (two to ten nucleotides) into the sense strands of the stem sequence at 1 to 100 positions.

### Example 6: Confirmation of siRNA-directed sequence-specific methylation

In the Examples as described above, the sequence-specific methylation of a target gene promoter region, which was mediated by synthetic siRNAs or siRNAs expressed from an expression vector, was analyzed by methylation-specific PCR. In this Example, the present inventors used bisulfite sequencing, which can confirm siRNA-directed methylation at a sequence level. The bisulfite reagent used in the analysis converts unmethylated cytosine, but not methylated cytosine, to uracil. Accordingly, the methylation of cytosine can be assessed by recovering DNAs from cells into which siRNAs or the like have been introduced, reacting the DNAs with the bisulfite reagent, analyzing their sequences, and confirming the presence or absence of substituted uracil. Treatment with the bisulfite reagent was carried out using a CpGenome DNA-modification kit (Intergen; USA).

Specifically, as described in Example 1, synthetic siRNAs (siRNAs each corresponding to sites 1 to 10) targeted to E-cadherin promoter were each introduced into human breast cancer cells (MCF-7 cells) using Oligofectamine (Invitrogen; USA). The genomic DNAs were recovered 96 hours after introduction and treated with bisulfite reagent using a CpGenome DNA-modification kit (Intergen; USA). After incubation with the bisulfite reagent, the promoter DNA was amplified by PCR. The amplified promoter DNA was inserted into a plasmid vector using a TA cloning kit (Invitrogen; USA). The resulting plasmid vector was introduced into *Escherichia coli* for cloning. The cloned plasmids were sequenced to monitor methylation frequency.

Some results are shown in Fig. 7 (methylation was evaluated by sequencing plasmids from ten randomly selected *E. coli* clones; boxes corresponding to methylated nucleotides in the plasmids are shaded). The synthetic siRNAs targeted to E-cadherin promoter (each of sites 1 to 10; each independently introduced) were found to direct the methylation of CpG sequences in a sequence-specific manner. It was also found that, in addition to CpG sequences, some CpNG sequences (wherein, N represents any one of A, T, C, and G) were methylated. siRNA-directed methylation was detected not only in the experiment using cancer cells such as MCF-7 cells, but also in the experiment using normal mammary gland cells (Fig. 8). Furthermore, it was also revealed that these siRNAs not only directed the methylation of portions to which siRNA sequences bound, but also influenced (caused methylation in) adjacent regions (Fig. 9).

The above-described experiments using the bisulfite sequencing assay showed that synthetic siRNAs targeted to E-cadherin promoter induced sequence-specific methylation.

In addition, the bisulfite sequencing assay was also used to test whether DNA methylation in the erbB2 promoter was induced in a site-specific manner by the tRNA promoter-based expression vector for siRNA. The introduction of the expression vector and recovering of genomic DNAs were achieved by the same procedures as the above-described

### Examples.

The bisulfite sequencing assay showed that, like the synthetic siRNAs, the siRNAs expressed by the tRNA promoter induced methylation in a sequence-specific manner (Fig. 10A). Likewise, the siRNAs expressed by the U6 promoter were also found to induce sequence-specific methylation (Fig. 10B).

### Example 7: Analysis of the mechanism for the siRNA-directed methylation

siRNAs are known to induce methylation of Lys9 on histone H3 in plants and *Schizosaccharomyces* (Zilberman D, Cao X, Jacobsen SE.ARGONAUTE4 control of locus-specific siRNA accumulation and DNA and histone methylation. Science. 2003 Jan 31; 299(5607):716-9; Volpe TA, Kidner C, Hall IM, Teng G, Grewal SI, Martienssen RA.Regulation of heterochromatic silencing and histone H3 lysine-9 methylation by RNAi.Science. 2002 Sep 13; 297(5588):1833-7).

Thus, whether the above siRNAs targeted to E-cadherin or erbB2 promoter also induced the methylation of Lys9 on histone H3 in animal cells was also tested.

A mixture of siRNAs, each targeted to sites 1 to 10 of E-cadherin promoter, was introduced into MCF-7 cells. After 96 hours, the cells were harvested, treated with 1% formaldehyde, and then lysed in a lysis buffer. An antibody against methylated Lys9 of histone H3 was added to the lysate and gently mixed for eight hours. Protein Sepharose A was added to the mixture for the pull-down reaction. After elution with 200 µl of a solution containing 1% SDS and 0.1 M NaHCO₃, the eluted material was cross-linked using formaldehyde. Then, PCR was carried out using primers specific to E-cadherin promoter. The amplified products were electrophoresed and the resulting bands were stained using ethidium bromide for detection.

The above-described chromatin immunoprecipitation (ChIP) experiments using antibody against methylated Lys9 of histone H3 revealed that the methylation of Lys9 on histone H3 is induced by the siRNAs in a sequence-specific manner (Fig. 11). When the expression of histone methyltransferase (HMT) was suppressed by siRNA (sense: tatggaatattatcttgtaaa (SEQ ID NO: 69); antisense: tttacaagataatattccata (SEQ ID NO: 70)), the degrees of methylation of Lys9 on histone H3 and of the CpG sequence were markedly reduced (Fig. 11). When the expression of DNA methyltransferase (DNMT1) was suppressed by siRNA, the degree of CpG sequence methylation was markedly reduced, but Lys9 on histone H3 was normally methylated (Fig. 11). These findings suggest that siRNA induces the methylation of CpG sequences in the E-cadherin promoter region through Lys9 on histone H3.

### Example 8: RNA interference by long dsRNAs in animal cells

The experiments described herein showed that synthetic siRNAs and siRNAs expressed by tRNA promoters or U6 promoters suppress the expression of target genes at a transcriptional level by inducing the methylation of CpG and CpNG sequences in a sequence-specific manner. However, the siRNAs used in the experiments consisted of 21 to 30 nucleotides, and thus can be predicted to induce partial methylation resulting in only partial suppression of target gene expression at a transcriptional level. In general, CpG islands in gene promoter regions range from approximately 1kbp to several kbp in length. Various siRNAs covering the entire region are required to achieve efficient suppression of target gene expression at the transcriptional level. However, it is inefficient to construct many types of synthetic siRNAs or siRNA expression vectors.

Long dsRNAs commonly enhance non-specific suppression of gene expression by activating interferon-mediated signaling pathways. However, there are reports that long dsRNAs localized in the nucleus do not activate interferon-mediated signaling pathways.

Accordingly, to localize long dsRNAs in the nucleus, the present inventors conceived of inserting genes encoding long dsRNAs into beta globulin introns whose expression is driven by a PolII promoter (Fig. 12). The long dsRNAs are released from the transcripts expressed by the vectors via processing in the nucleus to cleave the introns. The dsRNAs are processed into short RNAs consisting of 21 to 25 nucleotides by endogenous Dicer-like ribonuclease III.

In fact, the present inventors constructed a vector pSV-BGI, in which a gene encoding a dsRNA consisting of 500 nucleotides targeted to erbB2 promoter was inserted into a beta globulin intron whose expression is driven by PolII promoter. In addition, they also constructed vectors in which the gene encoding dsRNA was inserted downstream of an expression vector for tRNA promotor or U6 promoter. Each vector was introduced into MCF-7 cells. 96 hours after introduction, genomic DNAs were recovered and analyzed for methylation in the erbB2 promoter region using the method described in Example 4.

The results showed that pSV-BGI induced more efficient and more extensive methylation than the tRNA promoter and U6 promoter (Fig. 13).

Western blotting was carried out using an antibody that specifically recognized erbB2 protein. Then, the amount of phosphorylated eIF2α relative to that of the internal control, β-actin, was estimated using the NIH Image program. It was revealed that erbB2 gene expression was markedly suppressed as a result of the methylation described above (Fig. 14).

The cytotoxicity caused by the introduction of each vector was also evaluated. The cytotoxicity of dsRNAs can be assessed based on the degree of eIF2α phosphorylation by dsRNA-dependent protein kinase (PKR). Thus, Western blotting was carried out using an antibody that specifically recognized phosphorylated eIF2α, and the amount of phosphorylated eIF2α relative to that of the internal control β-actin was estimated using the NIH Image program.

It was found that the vector pSV-BGI was not cytotoxic, while both the expression vectors for tRNA promoter- and U6 promoter-based expression vectors exhibited cytotoxicity (Fig. 15). Accordingly, the vector (pSV-BGI), in which a gene encoding a long dsRNA is inserted into a beta globulin intron whose expression is driven by the PolII promoter, clearly efficiently induced extensive methylation, resulting in transcriptional gene silencing.

### Industrial Applicability

The present inventors found for the first time that synthetic siRNAs and vector-based siRNAs can induce DNMT1-dependent RMDM in a sequence-specific manner in human cells. Thus, these siRNAs can sequence-specifically regulate gene silencing at a transcriptional level.

According to the present invention, the expression level of specific genes in mammalian cells can be regulated by siRNAs not only through disruption of cognate mRNAs, but also by suppression of transcription. The tRNA-shRNAs in the present invention may be useful as therapeutic agents.

## Claims

1. A DNA methylation-inducing agent that comprises a dsRNA targeted to a site comprising CpG or CpNG (where N is any one of A, T, C, and G) in a DNA in a mammalian cell.

2. A DNA methylation-inducing agent that comprises an expression vector comprising a DNA encoding a dsRNA targeted to a site comprising CpG or CpNG (where N is any one of A, T, C, and G) in a DNA in a mammalian cell.

3. The DNA methylation-inducing agent of claim 1 or 2, wherein the dsRNA consists of 30 nucleotides or less.

4. The DNA methylation-inducing agent of claim 1 or 2, wherein the dsRNA consists of 30 to 5,000 nucleotides.

5. The DNA methylation-inducing agent of any one of claims 1 to 4, wherein the dsRNA comprises a hairpin structure.

6. The DNA methylation-inducing agent of claim 2, wherein the vector further encodes Dicer.

7. The DNA methylation-inducing agent of claim 2, wherein an exon-intron-exon cassette is operably linked downstream of a promoter and the DNA encoding the dsRNA is inserted into an intron.

8. The DNA methylation-inducing agent of claim 7, wherein the exon-intron-exon cassette is derived from an immunoglobulin gene.

9. The DNA methylation-inducing agent of any one of claims 2 to 7, wherein the DNA encoding the dsRNA is operatively linked to a PolI or PolII promoter in the expression vector.

10. The DNA methylation-inducing agent of claim 9, wherein the PolIII promoter is any one of tRNA promoter, U6 promoter, and H1 promoter.

11. The DNA methylation-inducing agent of claim 10, wherein the PolII promoter is any one of SV40 promoter, CMV promoter, and SRα promoter.

12. The DNA methylation-inducing agent of any one of claims 2 to 11, wherein the expression vector is a viral expression vector.

13. The DNA methylation-inducing agent of any one of claims 2 to 12, wherein the viral expression vector is any one of retrovirus, adenovirus, and lentivirus.

14. The DNA methylation-inducing agent of any one of claims 2 to 13, wherein the DNA encoding the dsRNA is operatively linked to a promoter comprising a tetracycline operator sequence (TetO) in the expression vector.

15. A DNA methylation method that comprises the step of introducing the methylation-inducing agent of any one of claims 1 to 14 into a mammalian cell.

16. The DNA methylation method of claim 15, wherein the mammal is human.

17. A gene expression-suppressing agent that comprises a dsRNA targeted to a site comprising CpG or CpNG (wherein, N is any one of A, T, C, and G) in a gene promoter in a mammalian cell.

18. A gene expression-suppressing agent that comprises an expression vector encoding a dsRNA targeted to a site comprising CpG or CpNG (where N is any one of A, T, C, and G) in a gene promoter in a mammalian cell.

19. The gene expression-suppressing agent of claim 17 or 18, wherein the types of dsRNA differ and the each of the dsRNAs target a different site comprising CpG or CpNG (where N represents any one of A, T, C, and G).

20. The gene expression-suppressing agent of any one of claims 17 to 19, wherein the dsRNA consists of 30 nucleotides or less.

21. The gene expression-suppressing agent of any one of claims 17 to 19, wherein the dsRNA consists of 30 to 5,000 nucleotides.

22. The gene expression-suppressing agent of any one of claims 17 to 21, wherein the dsRNA comprises a hairpin structure.

23. The gene expression-suppressing agent of claim 18, wherein the vector further encodes Dicer.

24. The gene expression-suppressing agent of claim 18, wherein an exon-intron-exon cassette is operably linked downstream of a promoter and the DNA encoding the dsRNA is inserted into the intron.

25. The gene expression-suppressing agent of claim 18, wherein the exon-intron-exon cassette is derived from an immunoglobulin gene.

26. The gene expression-suppressing agent of any one of claims 18 to 25, wherein the DNA encoding the dsRNA is operatively linked to a PolI or PolII promoter in the expression vector.

27. The gene expression-suppressing agent of claim 26, wherein the PolIII promoter is any one of tRNA promoter, U6 promoter, and H1 promoter.

28. The gene expression-suppressing agent of claim 26, wherein the PolII promoter is any one of SV40 promoter, CMV promoter, and SRα promoter.

29. The gene expression-suppressing agent of any one of claims 18 to 28, wherein the expression vector is a viral expression vector.

30. The gene expression-suppressing agent of claim 29, wherein the viral expression vector is any one of retrovirus, adenovirus, and lentivirus.

31. The gene expression-suppressing agent of any one of claims 18 to 30, wherein the DNA encoding the dsRNA is operatively linked to a promoter comprising a tetracycline operator sequence (TetO) in the expression vector.

32. The gene expression-suppressing agent of claims 17 to 31, wherein the gene is a disease-associated gene whose expression is involved in a disease.

33. The gene expression-suppressing agent of claim 32, wherein the disease is a tumor.

34. The gene expression-suppressing agent of claim 33, wherein the gene is erbB2.

35. A cell proliferation-suppressing agent that comprises the gene expression-suppressing agent of claim 34 as an active ingredient.

36. A gene expression-suppressing method that comprises the step of introducing the gene expression-suppressing agent of any one of claims 18 to 34 into a cell.

37. The gene expression-suppressing method of claim 36 that further comprises the step of introducing dsRNAs targeted to a coding region of a gene into a cell.

38. The gene expression-suppressing method of claim 36 or 37, wherein the mammalian cell is derived from a human.
